# EUROPEAN PATENT SPECIFICATION

(11) **EP 4 103 265 B1**
(45) Date of publication and mention of the grant of the patent: **24.12.2025**
(21) Application number: 21710155.9
(22) Date of filing: 12.02.2021
(51) Int. Cl.: A61N 1/36, A61N 1/05

(54) **STIMULATION ELECTRODE ASSEMBLIES AND SYSTEMS FOR TREATING SLEEP DISORDERED BREATHING**
STIMULATIONSELEKTRODEN-ANORDNUNGEN UND SYSTEME ZUR BEHANDLUNG VON ATEMSTÖRUNGEN IM SCHLAF
ENSEMBLES ÉLECTRODES DE STIMULATION ET SYSTÈMES DE TRAITEMENT DE TROUBLES RESPIRATOIRES DU SOMMEIL

(30) Priority: 14.02.2020 US 202062976466 P
(43) Date of publication of application: 21.12.2022
(73) Proprietor: Inspire Medical Systems, Inc., Golden Valley, MN 55416 (US)
(72) Inventor: VERZAL, Kevin, Golden Valley, Minnesota 55416 (US); OLSON, Nathan, Golden Valley, Minnesota 55416 (US); RONDONI, John, Golden Valley, Minnesota 55416 (US)
(74) Representative: Zimmermann & Partner Patentanwälte mbB
(86) International application number: PCT/US2021/017754
(87) International publication number: WO 2021/163402

(56) References cited:
- WO-A1-2013/075171
- US-A1- 2014 039 579
- US-A1- 2017 151 432

## Description

### Background

A significant portion of the population suffers from various forms of sleep apnea. In some patients, more than one type of sleep apnea may be exhibited. US 2014/0 039 579 A1 is related to a method for controlling energy delivery as a function of degree of coupling.

### Brief Description of the Drawings

FIG. 1A is a simplified top plan view of a stimulation electrode assembly in accordance with principles of the present disclosure.
FIG. 1B is a side view of the stimulation electrode assembly of FIG. 1A.
FIG. 2 is a schematic diagram from a lateral perspective showing the location and operation of muscles of the upper airway.
FIG. 3 is a schematic diagram showing the location of the hypoglossal nerve and its branches in relation to the anatomy of FIG. 2.
FIG. 4 is a schematic diagram from an anterior perspective showing a location the left and right hypoglossal nerves in relation to muscles of the upper airway.
FIG. 5A is a schematic diagram showing a possible implantation location in accordance with principles of the present disclosure of the stimulation electrode assembly of FIG. 1A relative to the anatomy of FIG. 4.
FIG. 5B is a schematic diagram representing a simplified profile view of the arrangement of FIG. 5A.
FIG. 5C is a schematic diagram showing a possible implantation location in accordance with principles of the present disclosure.
FIG. 6 is a simplified top plan view of another stimulation electrode assembly in accordance with principles of the present disclosure.
FIG. 7 is a simplified top plan view of another stimulation electrode assembly in accordance with principles of the present disclosure.
FIG. 8 is a simplified top plan view of another stimulation electrode assembly in accordance with principles of the present disclosure.
FIG. 9 is a simplified top plan view of another stimulation electrode assembly in accordance with principles of the present disclosure.
FIG. 10 is a simplified top plan view of another stimulation electrode assembly in accordance with principles of the present disclosure.
FIG. 11 is a simplified top plan view of another stimulation electrode assembly in accordance with principles of the present disclosure.
FIG. 12 is a simplified top plan view of another stimulation electrode assembly in accordance with principles of the present disclosure.
FIG. 13 is a simplified top plan view of another stimulation electrode assembly in accordance with principles of the present disclosure.
FIG. 14 is a simplified top plan view of another stimulation electrode assembly in accordance with principles of the present disclosure.
FIG. 15 is a simplified top plan view of another stimulation electrode assembly in accordance with principles of the present disclosure.
FIG. 16A is a simplified top plan view of another stimulation electrode assembly in accordance with principles of the present disclosure.
FIG. 16B is an enlarged, cross-sectional view of the stimulation electrode assembly of FIG. 16A, taken through one of the stimulation electrodes.
FIG. 16C is an enlarged, cross-sectional view of an alternative electrode arrangement useful with the stimulation electrode assembly of FIG. 16A.
FIG. 17A is a simplified top plan view of another stimulation electrode assembly in accordance with principles of the present disclosure.
FIG. 17B is a simplified top plan view of another stimulation electrode assembly in accordance with principles of the present disclosure
FIG. 17C is a simplified top plan view of another stimulation electrode assembly in accordance with principles of the present disclosure
FIG. 18 is a simplified perspective view of portions of an anatomy of an upper airway and illustrating methods in accordance with principles of the present disclosure.
FIG. 19 is a simplified cross-sectional view of an introducer in accordance with principles of the present disclosure, along with a stimulation electrode assembly.
FIG. 20 is a simplified cross-sectional view of another introducer in accordance with principles of the present disclosure, along with a stimulation electrode assembly.
FIG. 21A is a simplified end view of another stimulation electrode assembly in accordance with principles of the present disclosure.
FIG. 21B is a side view of the stimulation electrode assembly of FIG. 21A.
FIG. 22 is a schematic illustration of a stimulation electrode assembly implanted relative to two nerves in accordance with principles of the present disclosure, along with an implantable pulse generator connected to the stimulation electrode assembly.
FIG. 23A is a block diagram schematically representing an example control portion.
FIG. 23B is a diagram schematically representing at least some examples different modalities of the control portion of FIG. 23A.
FIG. 23C is a block diagram schematically representing an example user interface.

### Detailed Description

The invention provides a system for treating sleep disordered breathing according to claim 1. Embodiments of the invention are provided in the dependent claims. For better understanding of the invention, the present disclosure provides also further examples, not falling under the scope of the claims. In particular, the disclosed methods are not claimed . In the following detailed description, reference is made to the accompanying drawings which form a part hereof, and in which is shown by way of illustration specific examples in which the disclosure may be practiced. It is to be understood that other examples may be utilized and structural or logical changes may be made without departing from the scope of the present disclosure. The following detailed description, therefore, is not to be taken in a limiting sense. It is to be understood that features of the various examples described herein may be combined, in part or whole, with each other, unless specifically noted otherwise.

At least some examples of the present disclosure are directed to implantable stimulation electrode assemblies, useful, for example, with implantable systems for delivering stimulation therapy to a patient, along with methods of implanting such stimulation electrode assemblies and methods of delivering therapy. In some non-limiting examples, the stimulation electrode assemblies are configured for selectively delivering stimulation energy to discrete nerves/nerve segments, for example on a bilateral basis. In some embodiments, a single stimulation electrode assembly of the present disclosure can selectively affect discrete, bilateral nerves/nerve segments (e.g., nerve trunk, ends of nerve fibers, etc.), such as the right and left hypoglossal nerves. In related embodiments, the single stimulation electrode assembly can be implanted through a single incision (e.g., at or near a side of the patient's chin) and located so as to extend across the patient's mid-line.

At least some examples of the assemblies, systems and methods of the present disclosure are directed to sleep disordered breathing (SDB) therapy, such as obstructive sleep apnea (OSA) therapy, which may comprise monitoring, diagnosis, and/or stimulation therapy. However, in other examples, the assemblies, systems and methods of the present disclosure are used for other types of therapy, including, but not limited to, neurostimulation or cardiac therapy. In some embodiments, such other implementations include therapies, such as but not limited to, central sleep apnea, complex sleep apnea, cardiac disorders, pain management, seizures, deep brain stimulation, and respiratory disorders.

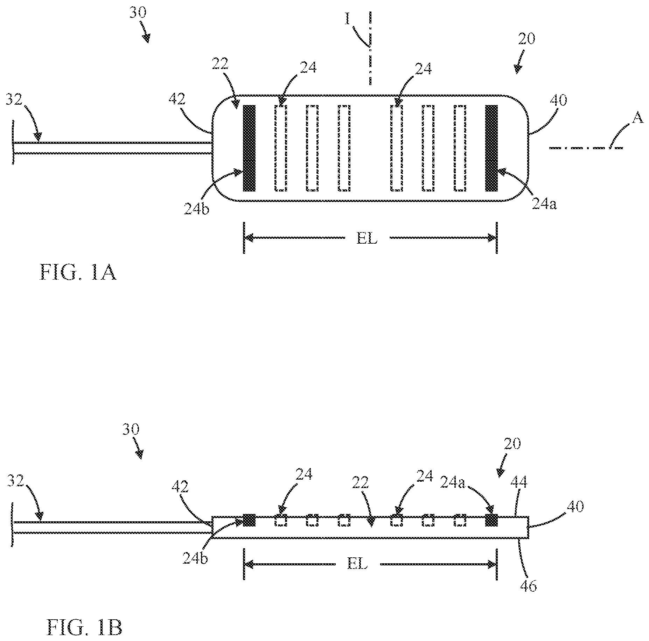

These examples, and additional examples, are further described in association with at least FIGS. 1-23C.

One example of a stimulation electrode assembly 20 in accordance with principles of the present disclosure is shown in simplified form in FIGS. 1A and 1B. The stimulation electrode assembly 20 includes a support body 22 and a plurality or array of stimulation electrodes 24. As a point of reference, in some non-limiting examples, the stimulation electrode assemblies of the present disclosure can be provided as part of a stimulation lead; thus, in the views of FIGS. 1A and 1B, the stimulation electrode assembly 20 is optionally provided as part of a stimulation lead 30 that further includes a lead body 32. The lead body 32 provides or carries wires (not shown) electrically connected to the stimulation electrodes 24, and is of sufficiently flexible construction and length for placement within a patient's body and for coupling to an implanted generator device (not shown) or the like as is known in the art to deliver electrical energy to the stimulation electrodes 24. In other embodiments, energy can be provided to the stimulation electrodes 24 by other techniques that may not require the lead body 32.

The support body 22 is configured to maintain the stimulation electrodes 24 (as well as other optional electrical components) in an electrically isolated manner, and is formed of a biocompatible material appropriate for implantation into the human body. The support body 22 may be flexible to increase patient comfort and allow for adaptation to varying patient anatomy. For example, the support body 22 can be configured to exhibit sufficient malleability or flexibility to conform to a general shape of a targeted implant site and optionally to further provide gentle pressure against tissue of the target site to maintain nerve contact. As described in greater detail below, the support body 22 can form or carry one or more features that facilitate one or both of delivery/implantation and fixation. Regardless, a form factor or foot print or shape of the support body 22 defines a first end 40 opposite a second end 42, and a top face 44 opposite a bottom face 46. With optional embodiments in which the lead body 32 is provided, the first end 40 can be considered a leading end (e.g., as the first end 40 is opposite the lead body 32) of the electrode assembly 20, and the second end 42 can be considered a trailing end. As revealed by FIG. 1A, the support body 22 can have an elongated shape that defines a length between the first and second ends 40, 42, and a width perpendicular to the length (a thickness of the support body 22 is evident in FIG. 1B). The length can be greater than the width, and a central major axis A of the shape of the support body 22 is defined along or relative to the length. A central minor axis I of the support body 22 is defined perpendicular to the central major axis A, mid-way between the first and second ends 40, 42. While the view of FIG. 1A implicates the support body 22 as having a paddle or paddle-like shape, other shapes are also acceptable as described in greater detail below. Similarly, while the view of FIG. 1B may implicate the support body 22 as having a generally linear shape in longitudinal extension, other shapes are also acceptable. For example, the support body 22 can have a pre-formed or pre-defined U-like shape, W-like shape, or other complex shape. In related optional embodiments, in addition to a pre-formed or pre-defined shape, the support body 22 can exhibit sufficient flexibility or "springiness" to slightly deflect when implanted against native anatomy or tissue.

Each of the stimulation electrodes 24 are formed of an electrically conductive material appropriate for delivering stimulation energy within the human body. The stimulation electrodes 24 can have the elongated (e.g., rectangular), block-like construction implicated by FIGS. 1A and 1B; in other embodiments, one or more or all of the stimulation electrodes 24 can have other shapes (e.g., square, cylinder, etc.) or constructions (e.g., akin to a wire, a coil, etc.). While each of the stimulation electrodes 24 are shown in FIG. 1A as having a substantially identical shape and orientation relative to the form factor or foot print of the support body 22, in other embodiments, various ones of the stimulation electrodes 24 can have differing shapes or orientations as described in greater detail below. Regardless, the stimulation electrodes 24 are arranged along the support body 22 so as to provide an exposed surface (from which stimulation energy is emitted) at or relative to the top face 44 of the support body 22. The stimulation electrodes 24 are encapsulated and electrically isolated from one another by the support body 22 and otherwise not exposed relative to the bottom face 46. Though not shown in the view of FIG. 1B, individual, electrically isolated wire(s) can extend from each of the stimulation electrodes 24 within a thickness of the support body 22.

The stimulation electrode assemblies of the present disclosure have at least two stimulation electrodes, but can have any number of electrodes greater than two. In some embodiments, a case of the energy source connected to the stimulation electrode assembly (e.g., a case of an implantable power generator (IPG)) can act as an electrode. As labeled for the example stimulation electrode assembly 20 of FIGS. 1A and 1B, the plurality of stimulation electrodes 24 includes a first end electrode 24a and a second end electrode 24b. The first end electrode 24a is considered to be the electrode most proximate the first end 40 of the support body 22, and the second end electrode 24b is considered to be the electrode most proximate the second end 42 of the support body 22. Alternatively stated, the first and second end electrodes 24a, 24b are located at opposite sides of the central minor axis I; the first end electrode 24a is the electrode located at a greatest distance from the central minor axis I in a first direction of the central major axis A as compared to all other electrodes, and the second end electrode 24b is the electrode located at a greatest distance from the central minor axis I in a second, opposite direction of the central major axis A. Thus, and consistent with the explanations above, the first end electrode 24a can be viewed as the leading electrode of the plurality of electrodes 24, and the second end electrode 24b can be viewed as the trailing electrode of the plurality of stimulation electrodes 24. Any number of stimulation electrodes 24 can be intermediately located between the first and second end electrodes 24a, 24b (several of which are shown with dashed lines). The stimulation electrodes 24 can all have a similar shape, or various ones of the stimulation electrodes 24 can exhibit different shapes. Regardless of the number, shape, orientation, and configuration of the stimulation electrodes 24 provided with the simulation electrode assembly 20, a distance between the opposing, first and second end electrodes 24a, 24b serves as an effective length EL of the stimulation electrode assembly 20. The stimulation electrode assembly 20 is configured such that the effective length EL corresponds with (e.g., approximates or exceeds) an expected span dimension of an anatomical target site upon final implant, for example the expected span dimension of a bilateral anatomical target site, as described below.

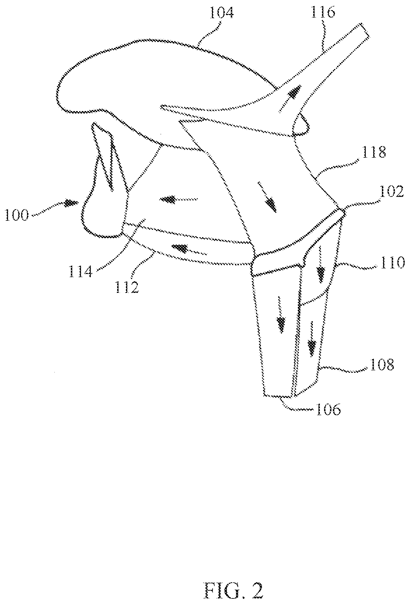

For example, in some non-limiting embodiments, the stimulation electrode assemblies, and corresponding systems and methods, of the present disclosure are configured or formatted to permit selective stimulation of bilateral nerves to treat SDB. By way of background, FIG. 2 is a simplified representation of muscle structures of a human adult upper airway provided at a lower jaw, together with the directional effects of their contractions on various airway structures. Anatomy of the lower jaw can be described with reference to a symphysis of mandible 100, a hyoid bone 102 and tongue 104. A sternohyoid muscle 106 extends upward from the sternum and clavicle (not shown) and is attached to the hyoid 102. A sternothyroid muscle 108 extends upward from the sternum and cartilage of the first rib (not shown) and is attached to the thyroid cartilage (not shown). A thyrohyoid muscle 110 extends from the thyroid cartilage, akin to an extension of the sternohyoid muscle 108, and upward into attachment with the hyoid 102. The sternohyoid, sternothyroid and thyrohyoid muscles 106-110 act to depress the larynx and hyoid 102.

A geniohyoid muscle 112 extends from the inner side of the symphysis of the lower jaw 100 to the hyoid 102. The geniohyoid muscle 112 serves as an elevator muscle for the hyoid 102 and the base of the tongue 104. A genioglossus muscle 114 is a fan-shaped, extrinsic pharyngeal muscle connecting the base of the tongue 104 to the chin, and has points of attachment with the lower jaw 100, the hyoid 102 and the tongue 104. The genioglossus muscle 114, by means of its posterior fibers, functions to draw the base of the tongue 104 forward so as to protrude the apex of the tongue 104 from the mouth. A styloglossus muscle 116 extends from the styloid process (not shown) to the tongue 104, and serves to draw the tongue 104 upward and backward. A hyoglossus muscle 118 extends from the hyoid 102 to the tongue 104, and is thin and quadrilateral in shape. The hyoglossus muscle 118 functions to retract the tongue 104 and to depress the tongue 104 at its sides so as to render the tongue 104 convex from side to side. The genioglossus, styloglossus and hyoglossus muscles 114-118 are extrinsic muscles of the tongue 104.

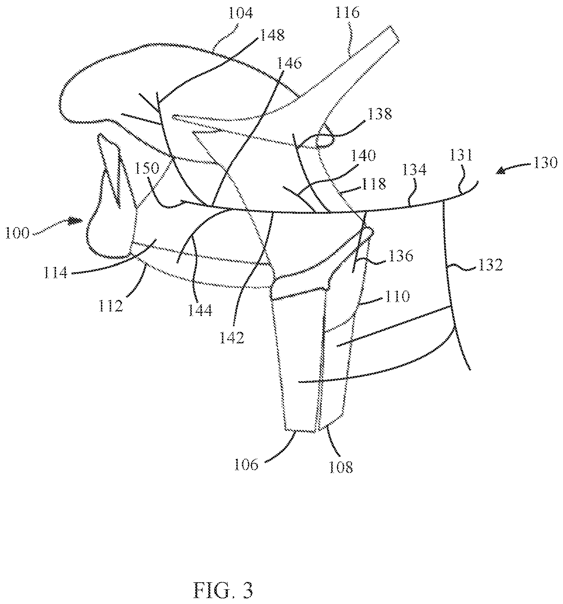

As shown schematically in FIG. 3, the muscles indicated above are innervated by a hypoglossal nerve 130. The hypoglossal nerve 130 includes a proximal main trunk 131 that divides into an ansa cervicalis branch 132 and a first medial nerve trunk 134. The ansa cervicalis branch 132 innervates the sternothyroid muscle 108. The first medial nerve trunk 134 includes a first branch 136 innervating the thyrohyoid muscle 110, a second branch 138 innervating the styloglossus muscle 116, and a third branch 140 innervating the hyoglossus muscle 118. An extension of the first medial nerve trunk 134 is a second medial nerve trunk 142 that has a branch 144 innervating the geniohyoid muscle 112. An extension of the second medial nerve trunk 142 is a third medial nerve trunk 146 that includes a first branch 148 innervating the muscles of the tongue 104, and a second branch 150 innervating the genioglossus muscle 114. As a point of reference, the order of the nerve branches reflected by FIG. 3 is not necessarily the same across all subjects.

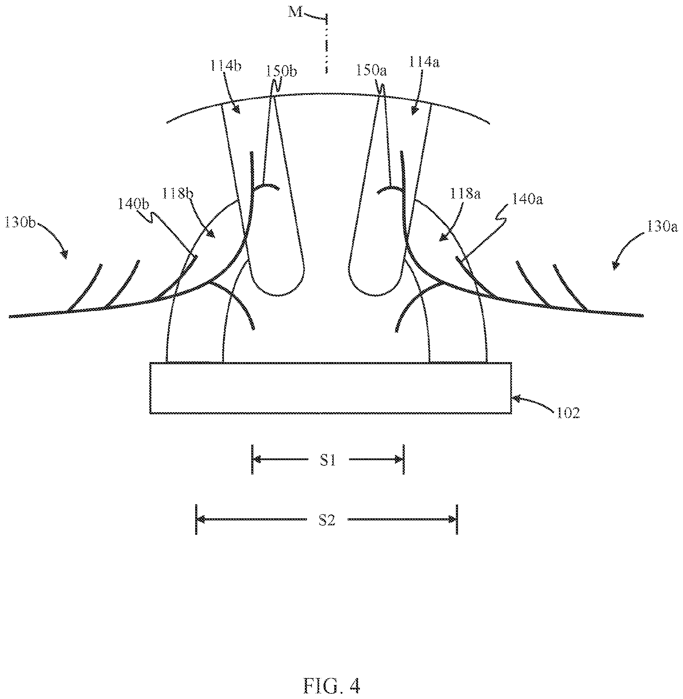

It will be understood that the views of FIGS. 2 and 3 omit various other anatomy so as to better represent the hypoglossal nerve 130. For example, a mylohyoid muscle (as a paired muscle) runs from the mandible (not shown) to the hyoid bone 102, and extends at least partially over various other anatomy, such as partly over the genioglossus muscle 114. Further, various ones of the muscles discussed above can be viewed as having discrete sections at opposite sides of the patient's medial plane or mid-line (and thus can be considered as bilateral). For example, the simplified representation of FIG. 4 identifies a mid-line or medial plane M of the patient, and reflects the genioglossus muscle generally arising from the hyoid 102 as genioglossus muscle sections 114a, 114b at opposite sides of the mid-line M (e.g., the section 114a can also be considered as a left genioglossus muscle and the section 114b as a right genioglossus muscle (where the terms "left" and "right" are used in reference to a perspective of the patient)). As a point of reference, only about 80% of the genioglossus muscle goes to the hyoid bone 102. Similarly, the hyoglossus muscle arises from the hyoid 102 as hyoglossus muscle sections 118a, 118b at opposite sides of the mid-line M (e.g., the section 118a can also be considered as a left hyoglossus muscle and the section 118b as a right hyoglossus muscle). These, and other, discrete muscles or muscle segments are separately innervated by discrete or separate nerves. For example, the left genioglossus muscle 114a and the left hyoglossus muscle 118a are innervated by a first or left hypoglossal nerve 130a, and the right genioglossus muscle 114b and the right hyoglossus muscle 118b are innervated by a second or right hypoglossal nerve 130b. The hypoglossal nerves 130a, 130b are thus situated (at least relative to the perspective of FIG. 4) at opposite sides of the mid-line M. The left and right hypoglossal nerves 130a, 130b extend anteriorly from opposite sides of the mid-line M, with the third medial nerve trunk second branch 150a of the left hypoglossal nerve 130a innervating the left genioglossus muscle 114a, and the third medial nerve trunk second branch 150b of the right hypoglossal nerve 130b innervating the right genioglossus muscle 114b. Similarly, the first medial nerve trunk third branch 140a of the left hypoglossal nerve 130a innervates the left hyoglossus muscle 118a, and the first medial nerve trunk third branch 140b of the right hypoglossal nerve 130b innervates the right hyoglossus muscle 118b. The left and right hypoglossal nerves 130a, 130b, and in particular the corresponding bilateral branches 140a, 140b and 150a, 150b, are discrete or separate from one another. As a point of reference, FIG. 4 further illustrates a branch of each of the hypoglossal nerves 130a, 130b extending to the geniohyoid muscle (not shown).

In some embodiments of the present disclosure, the stimulation electrode assemblies have a form factor (or size and shape) appropriate for providing stimulation energy to both of the left and right hypoglossal nerves 130a, 130b upon final implant, for example in a region of the third medial nerve trunk second branch 150a of the left hypoglossal nerve 130a and the third medial nerve trunk second branch 150b of the right hypoglossal nerve 130b, in a region of the first medial nerve trunk third branch 140a of the left hypoglossal nerve 130a and the first medial nerve trunk third branch 140b of the right hypoglossal nerve 130b, etc. These, and other, corresponding or bilateral regions can collectively define a target site span distance extending across the mid-line M. For example, FIG. 4 identifies a first span distance S1 that encompasses both the third medial nerve trunk second branch 150a of the left hypoglossal nerve 130a and the third medial nerve trunk second branch 150b of the right hypoglossal nerve 130b; a second span distance S2 is also identified that encompasses both the first medial nerve trunk third branch 140a of the left hypoglossal nerve 130a and the first medial nerve trunk third branch 140b of the right hypoglossal nerve 130b. Other span distances across the mid-line M and encompassing at least a portion (e.g., a branch, nerve endings, etc.) of two discrete nerves of interest can also be identified.

With additional reference to FIGS. 1A and 1B, the effective length **EL** can be selected in accordance with the span distance (e.g., S1, S2, etc.). For example, in some embodiments the stimulation electrode assembly 20 is configured for bilateral applications, acting upon both the left and right hypoglossal nerves 130a, 130b in or near a region of innervation with the left and right genioglossus muscles 114a, 114b. With these and related embodiments, the effective length EL will approximate or be greater than the expected first span distance S1 (in a human adult, the identified first span distance S1 is typically on the order of 3 cm). In addition or alternatively, the stimulation electrode assembly 20 can be configured to act upon the left and right hypoglossal nerves 130a, 130b in or near a region of innervation with the left and right hyoglossus muscles 118a, 118b. With these and related embodiments, the effective length EL will approximate or be greater than the expected second span distance S2 (in a human adult, the identified second span distance S2 is typically on the order of 5 cm). Other anatomical span distances can also be addressed by the selected effective length EL, and the present disclosure is not limited to stimulation of the left and right hypoglossal nerves 130a, 130b. For example, the space distance can be selected to encompass ends (nerve fiber ends) of the left and right hypoglossal nerves 130a, 130b.

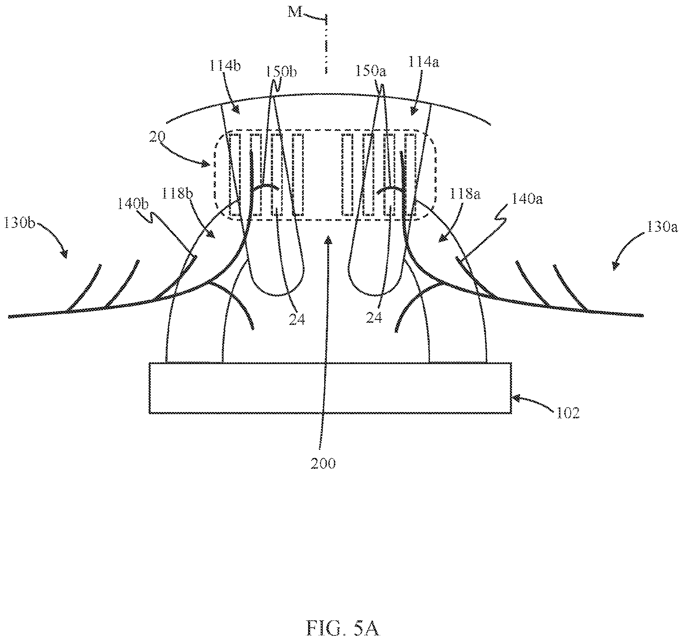

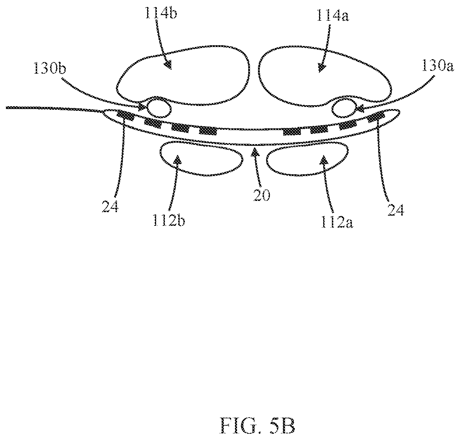

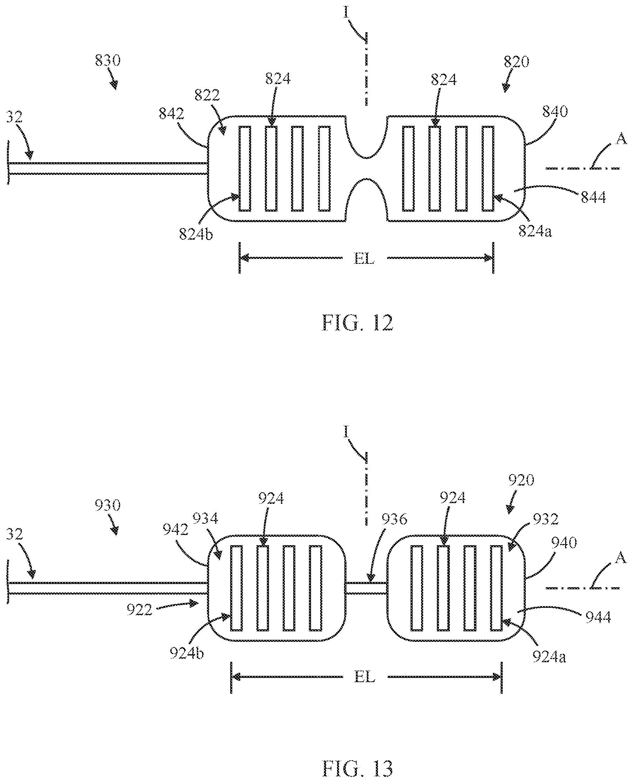

Some methods of the present disclosure for delivering the stimulation electrode assembly 20 to a desired target site are described in greater detail below. One possible final implant arrangement is identified at 200 in FIG. 5A. As shown, the stimulation electrode assembly 20 extends across the mid-line M of the patient, and provides at least one electrode 24 arranged to affect the left hypoglossal nerve 130a (e.g., the third medial nerve trunk second branch 150a of the left hypoglossal nerve 130a) and at least one other electrode 24 arranged to affect the right hypoglossal nerve 130b (e.g., the third medial nerve trunk second branch 150b of the right hypoglossal nerve 130b), for example on a bilateral basis. By providing the opposing end electrodes 24a, 24b (FIGS. 1A and 1B) at the effective length **EL** (FIGS. 1A and 1B) that corresponds with (e.g., is greater than) the expected span distance S1 (FIG. 4), at least one electrode 24 will be correctly located to best interact with the left and right hypoglossal nerves 130a, 130b. The additional stimulation electrodes between the end electrodes 24a, 24b allow for stimulation of the right and/or left hypoglossal nerves accounting for variation in patient anatomy and implant location. FIG. 5B represents one possible profile view of the final implant arrangement of FIG. 5A. As shown, the stimulation electrode assembly 20 can be implanted between the left and right genioglossus muscle sections 114a, 114b and the left and right geniohyoid muscle sections 112a, 112b. At least one of the electrodes 24 is arranged to affect the left hypoglossal nerve 130a, and another one of the electrodes 24 is arranged to affect the right hypoglossal nerve 130b. The electrodes 24 may be slightly spaced from the corresponding nerve 130a, 130b or may be in contact with the corresponding nerve 130a, 130b. Other target sites are also acceptable, and the present disclosure is not limited to stimulation of the left and right hypoglossal nerves 130a, 130b. As a point of reference, with the non-limiting example of FIG. 5B, the support body 22, and thus the stimulation electrode assembly 20 as a whole, is illustrated as having or assuming a generally uniform, slightly U-like shape upon final implant. This shape can be a pre-formed attribute of the support body 22. In other embodiments, the support bodies of the present disclosure can be configured (e.g., material, size, shape, etc.) to conform to various curvatures effected by native anatomy of the target site (e.g., can conform to curvature(s) of the genioglossus muscle section(s) 114a, 114b, the geniohyoid muscle section(s) 112a, 112b, etc.). This, in turn, can cause the stimulation electrode assembly 20 to have more a "W" or W-like shape (or other complex shape) upon final implant, for example. Some non-limiting examples of stimulation electrode assembly configurations that may be appropriate for better conforming to muscle profiles are described below with respect to FIGS. 12-14. The support bodies and stimulation electrode assemblies of the present disclosure optionally can have or exhibit sufficient malleability or flexibility to maintain good nerve contact upon final implant to a particular native anatomy target site. In other non-limiting examples, the support bodies and stimulation electrode assemblies of the present disclosure can have a pre-formed or pre-defined shape, and exhibit sufficient flexibility to provide gentle pressure against native anatomy tissue so as to maintain nerve contact. For example, relative to the target implant site implicated by FIG. 5B, the support body 22 could have a firm U or W shape (or other complex shape) that is more narrow, thereby allowing it to apply "upward pressure" and maintain more intimate nerve contact (e.g., a shape and/or "springiness" of the support body 22 is such that contact with the geniohyoid muscle sections 112a, 112b forces one or more of the electrodes 24 carried by the support body 22 into contact with the nerves 130a, 130b).

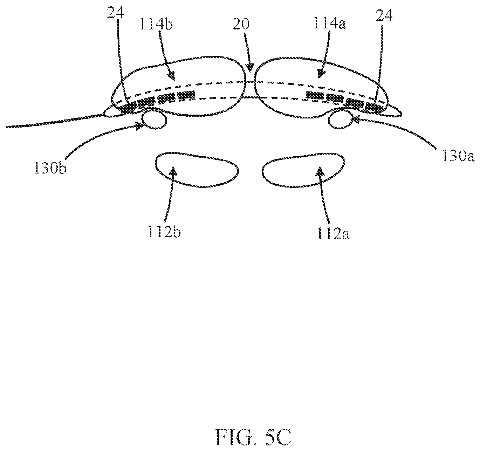

Any of the stimulation electrode assemblies, and corresponding leads, of the present disclosure can alternatively be configured and implanted such that the stimulation electrodes are positioned to stimulate or "capture" ends of the nerve fibers of interest (e.g., positioned near the nerve endings). By way of non-limiting example, with the arrangement of FIG. 5C, the electrode assembly 20 has been implanted in or near the genioglossus muscle base 114a, 114b, positioning selected ones of the stimulation electrodes 24 to deliver stimulating energy (from near the base of the genioglossus muscle 114a, 114b) near nerve endings of the left and right hypoglossal nerves 130a, 130b. One or more of the stimulation electrodes 24 may or may be within tissue of the genioglossus muscle 114a, 114b. Further, the support body 22 can optionally incorporate strain relief features (e.g., in region corresponding with the arrowhead of "20" in FIG. 5C) to accommodate relative motion between the left and right genioglossus muscle sections 114a, 114b. With the embodiments of FIG. 5C and related constructions, the electrode assembly 20 can be operated to not necessarily stimulate the genioglossus muscle directly, but instead to capture the ends of the nerve fibers by applying stimulation energy near the nerve endings at levels sufficient to stimulate the nerve ends.

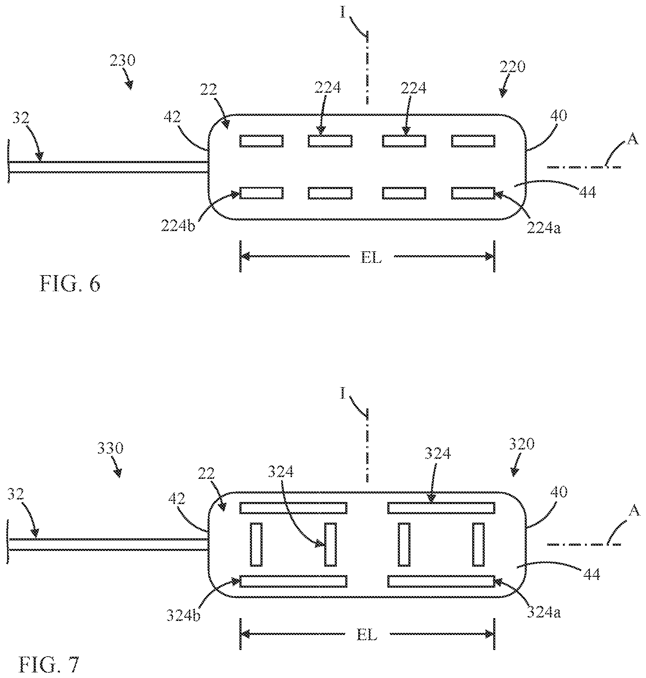

Returning to FIGS. 1A and 1B, the stimulation electrode assemblies of the present disclosure, for example the stimulation electrode assembly 20, can include the plurality of stimulation electrodes 24 arranged in a single row, with the elongated shape of each of the stimulation electrodes 24 being oriented perpendicular to the central major axis A of the support body 22. Other configurations or arrangements of the stimulation electrodes 24 relative to a shape or footprint of the support body 22 are also envisioned. For example, another stimulation electrode assembly 220 in accordance with principles of the present disclosure is shown in simplified form in FIG. 6. The stimulation electrode assembly 220 includes the support body 22 as described above and a plurality or array of stimulation electrodes 224. The stimulation electrode assembly 220 is optionally provided as part of a stimulation lead 230 that further includes the lead body 32 as described above.

Each of the stimulation electrodes 224 are formed of an electrically conductive material appropriate for delivering stimulation energy within the human body. The stimulation electrodes 224 can each have the elongated, block-like construction implicated by FIG. 6; in other embodiments, one or more or all of the stimulation electrodes 224 can have other shapes (e.g., square, cylinder, etc.) or constructions (e.g., akin to a wire, a coil, etc.). While each of the stimulation electrodes 224 are shown in FIG. 6 has having a substantially identical shape and orientation relative to the form factor or foot print of the support body 22, in other embodiments, various ones of the stimulation electrodes 224 can have differing shapes or orientations as described in greater detail below. Regardless, the stimulation electrodes 224 are arranged along the support body 22 so as to provide an exposed surface (from which stimulation energy is emitted) at or relative to the top face 44 of the support body 22. The stimulation electrodes 224 are encapsulated and electrically isolated from one another by the support body 22. In some embodiments, one or more of the stimulation electrodes 224 can be electrically common. Though not visible in the view of FIG. 6, individual, electrically isolated wire(s) can extend from each of the stimulation electrodes 224 within a thickness of the support body 22.

The stimulation electrodes 224 are arranged in a two row array across the support body 22, with the elongated shape of the each of the stimulation electrodes 224 oriented substantially parallel (i.e., within 5 degrees of a truly parallel relationship) to the major central axis A. Any other number of rows (greater than two) is also acceptable. The plurality of stimulation electrodes 224 includes a first end electrode 224a and a second end electrode 224b. The first end electrode 224a is the stimulation electrode most proximate the first end 40 of the support body 22, and the second end electrode 224b is the stimulation electrode most proximate the second end 42 of the support body 22. Thus, the first end electrode 224a can be viewed as the leading electrode of the plurality of stimulation electrodes 224, and the second end electrode 224b can be viewed as the trailing electrode of the plurality of stimulation electrodes 224. With embodiments in which the two or more rows of stimulation electrodes are aligned (e.g., as with the embodiment of FIG. 6), there may be two (or more) aligned, first or leading end electrodes 224a and/or to (or more aligned, second or trailing end electrodes 224b. Any number of stimulation electrodes 224 can be intermediately located between the first and second end electrodes 224a, 224b. Regardless of the number, shape, orientation, and configuration of the stimulation electrodes 224 provided with the simulation electrode assembly 220, a distance between the opposing, first and second end electrodes 224a, 224b serves as the effective length EL of the stimulation electrode assembly 220. Commensurate with the descriptions above, the stimulation electrode assembly 220 is configured such that the effective length EL corresponds with (e.g., approximates or exceeds) an expected span dimension of an anatomical target site upon final implant, for example the expected span dimension of a bilateral anatomical target site (e.g., and with additional reference to FIG. 4, the span distance S1 between the left and right hypoglossal nerves 130a, 130b in or near a region of corresponding innervation with the left and right genioglossus muscles 114a, 114b; the span distance S2 between the left and right hypoglossal nerves 130a, 130b in or near a region of corresponding innervation with the left and right hyoglossus muscles 118a, 118b; the span distance between nerve endings of the left and right hypoglossal nerves 130a, 130b; etc.).

While several embodiments have illustrated the stimulation electrodes associated with the stimulation electrode assembly as having a common orientation relative to the central major axis A, other configurations are also acceptable. For example, another stimulation electrode assembly 320 in accordance with principles of the present disclosure is shown in simplified form in FIG. 7. The stimulation electrode assembly 320 includes the support body 22 as described above and a plurality or array of stimulation electrodes 324. The stimulation electrode assembly 320 is optionally provided as part of a stimulation lead 330 that further includes the lead body 32 as described above.

Each of the stimulation electrodes 324 are formed of an electrically conductive material appropriate for delivering stimulation energy within the human body. The stimulation electrodes 324 can each have the elongated, block-like construction implicated by FIG. 7; in other embodiments, one or more or all of the stimulation electrodes 324 can have other shapes (e.g., square, cylinder, etc.) or constructions (e.g., akin to a wire, a coil, etc.). The stimulation electrodes 324 are arranged along the support body 22 so as to provide an exposed surface (from which stimulation energy is emitted) at or relative to the top face 44 of the support body 22. The stimulation electrodes 324 are encapsulated and electrically isolated from one another by the support body 22. In some embodiments, one or more of the stimulation electrodes 324 can be electrically common. Though not visible in the view of FIG. 7, individual, electrically isolated wire(s) can extend from each of the stimulation electrodes 324 within a thickness of the support body 22.

The stimulation electrodes 324 are symmetrically arranged relative to the central minor axis I, and include one or more stimulation electrodes oriented with the elongated shape thereof substantially parallel with the central major axis A (i.e., within 5 degrees of a truly parallel relationship) and one or more stimulation electrodes oriented with the elongated shape thereof substantially perpendicular to the central major axis A (i.e., within 5 degrees of a truly perpendicular relationship). Regardless, the plurality of stimulation electrodes 324 includes a first end electrode 324a and a second end electrode 324b. The first end electrode 324a is the stimulation electrode most proximate the first end 40 of the support body 22, and the second end electrode 324b is the stimulation electrode most proximate the second end 42 of the support body 22. Thus, the first end electrode 324a can be viewed as the leading electrode of the plurality of stimulation electrodes 324, and the second end electrode 324b can be viewed as the trailing electrode of the plurality of stimulation electrodes 324. With some embodiments (e.g., as with the embodiment of FIG. 7), there may be two (or more) aligned, first or leading end electrodes 324a and/or two (or more) aligned, second or trailing end electrodes 324b. Any number of stimulation electrodes 324 can be intermediately located between the first and second end electrodes 324a, 324b. Regardless of the number, shape, orientation, and configuration of the stimulation electrodes 324 provided with the simulation electrode assembly 320, a distance between the opposing, first and second end electrodes 324a, 324b serves as the effective length EL of the stimulation electrode assembly 320. Commensurate with the descriptions above, the stimulation electrode assembly 320 is configured such that the effective length EL corresponds with (e.g., approximates or exceeds) an expected span dimension of an anatomical target site upon final implant, for example the expected span dimension of a bilateral anatomical target site (e.g., and with additional reference to FIG. 4, the span distance S1 between the left and right hypoglossal nerves 130a, 130b in or near a region of corresponding innervation with the left and right genioglossus muscles 114a, 114b; the span distance S2 between the left and right hypoglossal nerves 130a, 130b in or near a region of corresponding innervation with the left and right hyoglossus muscles 118a, 118b; the span distance between nerve endings of the left and right hypoglossal nerves 130a, 130b; etc.).

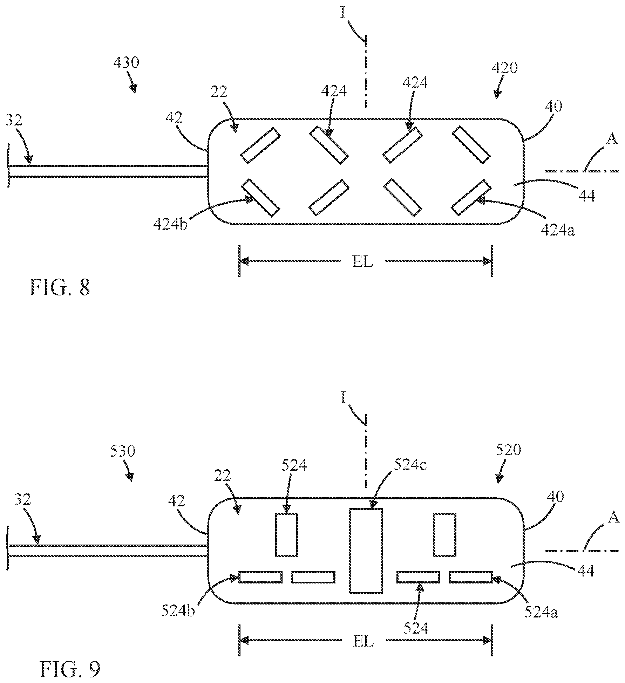

While several embodiments have illustrated the stimulation electrodes associated with the stimulation electrode assembly as having a substantially parallel or substantially perpendicular orientation relative to the central major axis A, other configurations are also acceptable. For example, another stimulation electrode assembly 420 in accordance with principles of the present disclosure is shown in simplified form in FIG. 8. The stimulation electrode assembly 420 includes the support body 22 as described above and a plurality or array of stimulation electrodes 424. The stimulation electrode assembly 420 is optionally provided as part of a stimulation lead 430 that further includes the lead body 32 as described above.

Each of the stimulation electrodes 424 are formed of an electrically conductive material appropriate for delivering stimulation energy within the human body. The stimulation electrodes 424 can each have the elongated, block-like construction implicated by FIG. 8; in other embodiments, one or more or all of the stimulation electrodes 424 can have other shapes (e.g., square, cylinder, etc.) or constructions (e.g., akin to a wire, a coil, etc.). The stimulation electrodes 424 are arranged along the support body 22 so as to provide an exposed surface (from which stimulation energy is emitted) at or relative to the top face 44 of the support body 22. The stimulation electrodes 424 are encapsulated and electrically isolated from one another by the support body 22. In some embodiments, one or more of the stimulation electrodes 424 can be electrically common. Though not visible in the view of FIG. 8, individual, electrically isolated wire(s) can extend from each of the stimulation electrodes 424 within a thickness of the support body 22.

A collective arrangement of the stimulation electrodes 424 relative to the central minor axis I can be symmetric, and can include one or more stimulation electrodes oriented with the elongated shape thereof non-parallel and non-perpendicular with the central major axis A (e.g., arranged at a 45 degree angle relative to the central major axis A). Regardless, the plurality of stimulation electrodes 424 includes a first end electrode 424a and a second end electrode 424b. The first end electrode 424a is the stimulation electrode most proximate the first end 40 of the support body 22, and the second end electrode 424b is the stimulation electrode most proximate the second end 42 of the support body 22. Thus, the first end electrode 424a can be viewed as the leading electrode of the plurality of stimulation electrodes 424, and the second end electrode 424b can be viewed as the trailing electrode of the plurality of stimulation electrodes 424. With some embodiments (e.g., as with the embodiment of FIG. 8), there may be two (or more) aligned, first or leading end electrodes 424a and/or to (or more) aligned, second or trailing end electrodes 424b. Any number of stimulation electrodes 424 can be intermediately located between the first and second end electrodes 424a, 424b. Regardless of the number, shape, orientation, and configuration of the stimulation electrodes 424 provided with the simulation electrode assembly 420, a distance between the opposing, first and second end electrodes 424a, 424b serves as the effective length EL of the stimulation electrode assembly 420. Commensurate with the descriptions above, the stimulation electrode assembly 420 is configured such that the effective length EL corresponds with (e.g., approximates or exceeds) an expected span dimension of an anatomical target site upon final implant, for example the expected span dimension of a bilateral anatomical target site (e.g., and with additional reference to FIG. 4, the span distance S1 between the left and right hypoglossal nerves 130a, 130b in or near a region of corresponding innervation with the left and right genioglossus muscles 114a, 114b; the span distance S2 between the left and right hypoglossal nerves 130a, 130b in or near a region of corresponding innervation with the left and right hyoglossus muscles 118a, 118b; the span distance between nerve endings of the left and right hypoglossal nerves 130a, 130b; etc.).

While several embodiments have illustrated the stimulation electrodes associated with the stimulation electrode assembly as having a substantially identically sized stimulation electrodes, other configurations are also acceptable. For example, another stimulation electrode assembly 520 in accordance with principles of the present disclosure is shown in simplified form in FIG. 9. The stimulation electrode assembly 520 includes the support body 22 as described above and a plurality or array of stimulation electrodes 524. The stimulation electrode assembly 520 is optionally provided as part of a stimulation lead 530 that further includes the lead body 32 as described above.

Each of the stimulation electrodes 524 are formed of an electrically conductive material appropriate for delivering stimulation energy within the human body. The stimulation electrodes 524 can each have the elongated, block-like construction implicated by FIG. 9; in other embodiments, one or more or all of the stimulation electrodes 524 can have other shapes (e.g., square, cylinder, etc.) or constructions (e.g., akin to a wire, a coil, etc.). The stimulation electrodes 524 are arranged along the support body 22 so as to provide an exposed surface (from which stimulation energy is emitted) at or relative to the top face 44 of the support body 22. The stimulation electrodes 524 are encapsulated and electrically isolated from one another by the support body 22. In some embodiments, one or more of the stimulation electrodes 524 can be electrically common. Though not visible in the view of FIG. 9, individual, electrically isolated wire(s) can extend from each of the stimulation electrodes 524 within a thickness of the support body 22.

A collective arrangement of the stimulation electrodes 524 relative to the central minor axis I can be symmetric, and can include one or more stimulation electrodes oriented with the elongated shape thereof substantially parallel with the central major axis and one more stimulation electrodes oriented with the elongated shape thereof substantially perpendicular to the central major axis A. Further, the stimulation electrodes 524 can define a pseudo-random array, and can include a large center electrode 524c that is common to both sides of the support body 22 (e.g., the center electrode extends along the central minor axis I). In some cases, the center electrode 524c can be utilized as part of the stimulation vector for both the right and left hypoglossal nerves. Regardless, the plurality of stimulation electrodes 524 includes a first end electrode 524a and a second end electrode 524b. The first end electrode 524a is the stimulation electrode most proximate the first end 40 of the support body 22, and the second end electrode 524b is the stimulation electrode most proximate the second end 42 of the support body 22. Thus, the first end electrode 524a can be viewed as the leading electrode of the plurality of stimulation electrodes 524, and the second end electrode 524b can be viewed as the trailing electrode of the plurality of stimulation electrodes 524. Any number of stimulation electrodes 524 can be intermediately located between the first and second end electrodes 524a, 524b. Regardless of the number, shape, orientation, and configuration of the stimulation electrodes 524 provided with the simulation electrode assembly 520, a distance between the opposing, first and second end electrodes 524a, 524b serves as the effective length EL of the stimulation electrode assembly 520. Commensurate with the descriptions above, the stimulation electrode assembly 520 is configured such that the effective length EL corresponds with (e.g., approximates or exceeds) an expected span dimension of an anatomical target site upon final implant, for example the expected span dimension of a bilateral anatomical target site (e.g., and with additional reference to FIG. 4, the span distance S1 between the left and right hypoglossal nerves 130a, 130b in or near a region of corresponding innervation with the left and right genioglossus muscles 114a, 114b; the span distance S2 between the left and right hypoglossal nerves 130a, 130b in or near a region of corresponding innervation with the left and right hyoglossus muscles 118a, 118b; the span distance between nerve endings of the left and right hypoglossal nerves 130a, 130b; etc.).

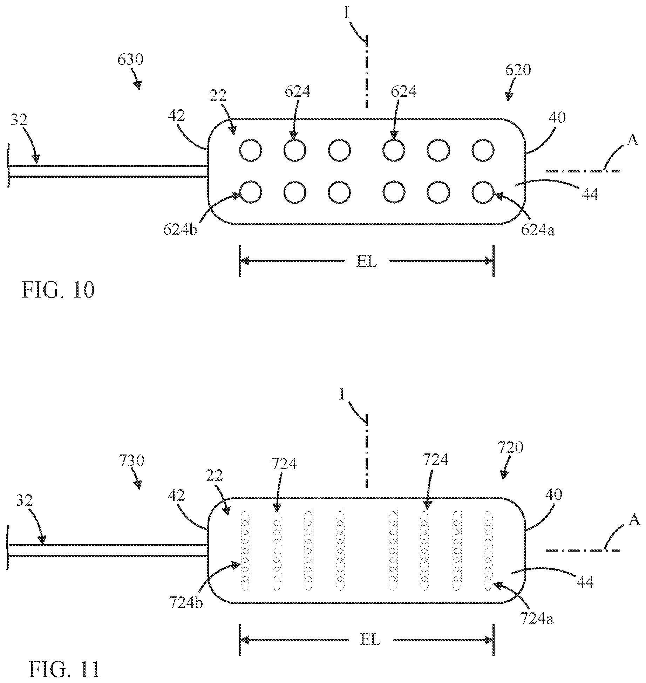

While several embodiments have illustrated the stimulation electrodes associated with the stimulation electrode assembly as having one or more rectangular shaped stimulation electrodes, other configurations are also acceptable. For example, another stimulation electrode assembly 620 in accordance with principles of the present disclosure is shown in simplified form in FIG. 10. The stimulation electrode assembly 620 includes the support body 22 as described above and a plurality or array of stimulation electrodes 624. The stimulation electrode assembly 620 is optionally provided as part of a stimulation lead 630 that further includes the lead body 32 as described above.

Each of the stimulation electrodes 624 are formed of an electrically conductive material appropriate for delivering stimulation energy within the human body. One or more or all of the stimulation electrodes 624 can have the circular shape implicated by FIG. 10; in other embodiments, one or more of the stimulation electrodes 624 can have other shapes (e.g., square, cylinder, etc.) or constructions (e.g., akin to a wire, a coil, etc.). The stimulation electrodes 624 are arranged along the support body 22 so as to provide an exposed surface (from which stimulation energy is emitted) at or relative to the top face 44 of the support body 22. The stimulation electrodes 624 are encapsulated and electrically isolated from one another by the support body 22. In some embodiments, one or more of the stimulation electrodes 624 can be electrically common. Though not visible in the view of FIG. 10, individual, electrically isolated wire(s) can extend from each of the stimulation electrodes 624 within a thickness of the support body 22.

A collective arrangement of the stimulation electrodes 624 relative to the central minor axis I can be symmetric, and includes a first end electrode 624a and a second end electrode 624b. The first end electrode 624a is the stimulation electrode most proximate the first end 40 of the support body 22, and the second end electrode 624b is the stimulation electrode most proximate the second end 42 of the support body 22. Thus, the first end electrode 624a can be viewed as the leading electrode of the plurality of electrodes 624, and the second end electrode 624b can be viewed as the trailing electrode of the plurality of stimulation electrodes 624. With some embodiments (e.g., as with the embodiment of FIG. 10), there may be two (or more) aligned, first or leading end electrodes 624a and/or to (or more) aligned, second or trailing end electrodes 624b. Any number of stimulation electrodes 624 can be intermediately located between the first and second end electrodes 624a, 624b. Regardless of the number, shape, orientation, and configuration of the stimulation electrodes 624 provided with the simulation electrode assembly 620, a distance between the opposing, first and second end electrodes 624a, 624b serves as the effective length EL of the stimulation electrode assembly 620. Commensurate with the descriptions above, the stimulation electrode assembly 620 is configured such that the effective length EL corresponds with (e.g., approximates or exceeds) an expected span dimension of an anatomical target site upon final implant, for example the expected span dimension of a bilateral anatomical target site (e.g., and with additional reference to FIG. 4, the span distance S1 between the left and right hypoglossal nerves 130a, 130b in or near a region of corresponding innervation with the left and right genioglossus muscles 114a, 114b; the span distance S2 between the left and right hypoglossal nerves 130a, 130b in or near a region of corresponding innervation with the left and right hyoglossus muscles 118a, 118b; the span distance between nerve endings of the left and right hypoglossal nerves 130a, 130b; etc.).

While several embodiments have illustrated the stimulation electrodes associated with the stimulation electrode assembly as having a solid or block-like construction, other configurations are also acceptable. For example, another stimulation electrode assembly 720 in accordance with principles of the present disclosure is shown in simplified form in FIG. 11. The stimulation electrode assembly 720 includes the support body 22 as described above and a plurality or array of stimulation electrodes 724. The stimulation electrode assembly 720 is optionally provided as part of a stimulation lead 730 that further includes the lead body 32 as described above.

Each of the stimulation electrodes 724 are formed of an electrically conductive material appropriate for delivering stimulation energy within the human body. One or more or all of the stimulation electrodes 724 can be a coiled wire as implicated by FIG. 11; in other embodiments, one or more of the stimulation electrodes 724 can have other shapes or constructions. The stimulation electrodes 724 are arranged along the support body 22 so as to provide an exposed surface (from which stimulation energy is emitted) at or relative to the top face 44 of the support body 22. The stimulation electrodes 724 are encapsulated and electrically isolated from one another by the support body 22. In some embodiments, one or more of the stimulation electrodes 724 can be electrically common. Though not visible in the view of FIG. 11, individual, electrically isolated wire(s) can extend from each of the stimulation electrodes 724 within a thickness of the support body 22.

A collective arrangement of the stimulation electrodes 724 relative to the central minor axis I can be symmetric, and includes a first end electrode 724a and a second end electrode 724b. The first end electrode 724a is the stimulation electrode most proximate the first end 40 of the support body 22, and the second end electrode 724b is the stimulation electrode most proximate the second end 42 of the support body 22. Thus, the first end electrode 724a can be viewed as the leading electrode of the plurality of stimulation electrodes 724, and the second end electrode 724b can be viewed as the trailing electrode of the plurality of stimulation electrodes 724. Any number of stimulation electrodes 724 can be intermediately located between the first and second end electrodes 724a, 724b. Regardless of the number, shape, orientation, and configuration of the stimulation electrodes 724 provided with the simulation electrode assembly 720, a distance between the opposing, first and second end electrodes 724a, 724b serves as the effective length EL of the stimulation electrode assembly 720. Commensurate with the descriptions above, the stimulation electrode assembly 720 is configured such that the effective length EL corresponds with (e.g., approximates or exceeds) an expected span dimension of an anatomical target site upon final implant, for example the expected span dimension of a bilateral anatomical target site (e.g., and with additional reference to FIG. 4, the span distance S1 between the left and right hypoglossal nerves 130a, 130b in or near a region of corresponding innervation with the left and right genioglossus muscles 114a, 114b; the span distance S2 between the left and right hypoglossal nerves 130a, 130b in or near a region of corresponding innervation with the left and right hyoglossus muscles 118a, 118b; the span distance between nerve endings of the left and right hypoglossal nerves 130a, 130b; etc.).

While several embodiments have illustrated the support body associated with the stimulation electrode assembly as having a relatively uniform perimeter shape, other configurations are also acceptable. For example, another stimulation electrode assembly 820 in accordance with principles of the present disclosure is shown in simplified form in FIG. 12. The stimulation electrode assembly 820 includes a support body 822 and a plurality or array of stimulation electrodes 824. The stimulation electrode assembly 820 is optionally provided as part of a stimulation lead 830 that further includes the lead body 32 as described above.

The support body 822 is akin to the support body 22 (FIG. 1) described above, and is configured to maintain the stimulation electrodes 824 (as well as other optional electrical components) in an electrically isolated manner. The support body 822 is formed of a biocompatible material appropriate for implantation into the human body. As described in greater detail below, the support body 822 can form or carry one or more features that facilitate one or both of implantation and fixation. Regardless, a form factor or foot print or shape of the support body 822 defines a first end 840 opposite a second end 842, and a top face 844 opposite a bottom face (hidden in the view). With optional embodiments in which the lead body 32 is provided, the first end 840 can be considered a leading end (e.g., as the first end 840 is opposite the lead body 32) of the stimulation electrode assembly 820, and the second end 842 can be considered a trailing end. A shape of the support body 822 defines a length between the first and second ends 840, 842, and a width perpendicular to the length. The length can be greater than the width, and a central major axis A of the shape of the support body 822 is defined along or relative to the length. A central minor axis I of the support body 822 is defined perpendicular to the central major axis A, mid-way between the first and second ends 840, 842.

With the non-limiting example of FIG. 12, the support body 822 can have or define a variable width, for example a decreased width at or near the central minor axis I (e.g., the hour glass shape of FIG. 12). With these and related embodiments, the thinned region of the support body 822 can exhibit enhanced flexibility as compared to other embodiments, with less shear force being imparted upon the support body 822 upon final implantation. The increased flexibility could accommodate relative motion between the right and left muscles during stimulation. Other shapes differing from the paddle shape and the hour glass shaped described above are also envisioned for the stimulation electrode assemblies of the present disclosure; for example, the support body can alternatively have a cylindrical shape, an oval shape, a butterfly shape, etc. The thinned region could be part of the support body 822, or could be a separate component that joins two discrete support body sections.

Each of the stimulation electrodes 824 are formed of an electrically conductive material appropriate for delivering stimulation energy within the human body. The stimulation electrodes 824 can assume any of the constructions of the present disclosure, and can, for example, be provided as elongated or rectangular block electrodes; in other embodiments, one or more of the stimulation electrodes 824 can have other shapes (e.g., square, cylinder, etc.) or constructions (e.g., akin to a wire, a coil, etc.). The stimulation electrodes 824 are arranged along the support body 822 so as to provide an exposed surface (from which stimulation energy is emitted) at or relative to the top face 844 of the support body 822. The stimulation electrodes 824 are encapsulated and electrically isolated from one another by the support body 822. In some embodiments, one or more of the stimulation electrodes 824 can be electrically common. Though not visible in the view of FIG. 12, individual, electrically isolated wire(s) can extend from each of the stimulation electrodes 824 within a thickness of the support body 822.

A collective arrangement of the stimulation electrodes 824 relative to the central minor axis I can be symmetric, and includes a first end electrode 824a and a second end electrode 824b. The first end electrode 824a is the stimulation electrode most proximate the first end 840 of the support body 822, and the second end electrode 824b is the stimulation electrode most proximate the second end 842 of the support body 822. Thus, the first end electrode 824a can be viewed as the leading electrode of the plurality of stimulation electrodes 824, and the second end electrode 824b can be viewed as the trailing electrode of the plurality of stimulation electrodes 824. Any number of stimulation electrodes 824 can be intermediately located between the first and second end electrodes 824a, 824b. Regardless of the number, shape, orientation, and configuration of the stimulation electrodes 824 provided with the simulation electrode assembly 820, a distance between the opposing, first and second end electrodes 824a, 824b serves as the effective length EL of the stimulation electrode assembly 820. Commensurate with the descriptions above, the stimulation electrode assembly 820 is configured such that the effective length EL corresponds with (e.g., approximates or exceeds) an expected span dimension of an anatomical target site upon final implant, for example the expected span dimension of a bilateral anatomical target site (e.g., and with additional reference to FIG. 4, the span distance S1 between the left and right hypoglossal nerves 130a, 130b in or near a region of corresponding innervation with the left and right genioglossus muscles 114a, 114b; the span distance S2 between the left and right hypoglossal nerves 130a, 130b in or near a region of corresponding innervation with the left and right hyoglossus muscles 118a, 118b; the span distance between nerve endings of the left and right hypoglossal nerves 130a, 130b; etc.).

Another stimulation electrode assembly 920 in accordance with principles of the present disclosure is shown in simplified form in FIG. 13. The stimulation electrode assembly 920 includes a support body 922 (referenced generally) and a plurality or array of stimulation electrodes 924. The stimulation electrode assembly 920 is optionally provided as part of a stimulation lead 930 that further includes the lead body 32 as described above.

The support body 922 is akin to the support body 22 (FIG. 1) described above, and is configured to maintain the stimulation electrodes 924 (as well as other optional electrical components) in an electrically isolated manner. With the non-limiting example of FIG. 13, the support body 924 is collectively defined by a plurality of discrete sections, such as a first support body section 932 and a second support body section 934. The support body sections 932, 934 are physically connected to one another by a joining element 936. In related embodiments, the support body 922 can be collectively defined by three (or more) discrete support body sections. The support body sections 932, 934 can be substantially identical in some embodiments, formed of a biocompatible material appropriate for implantation into the human body. As described in greater detail below, the support body 922 can form or carry one or more features that facilitate one or both of implantation and fixation. Regardless, a form factor or foot print or shape of the support body 922 defines a first end 940 opposite a second end 942, and a top face 944 opposite a bottom face (hidden in the view). With optional embodiments in which the lead body 32 is provided, the first end 940 can be considered a leading end (e.g., as the first end 940 is opposite the lead body 32) of the stimulation electrode assembly 920, and the second end 942 can be considered a trailing end. A shape of the support body 922 defines a length between the first and second ends 940, 942, and a width perpendicular to the length. The length can be greater than the width, and a central major axis A of the shape of the support body 922 is defined along or relative to the length. A central minor axis I of the support body 922 is defined perpendicular to the central major axis A, mid-way between the first and second ends 940, 942.

The joining element 936 can assume various forms, and in some embodiments is, or is akin to, a cable or ribbon. The joining element 936 encases or carries wiring (not shown) extending to and from the stimulation electrodes 924 of the first support body section 932. As compared to a configuration of the support body sections 932, 934, the joining element 936 can exhibit enhanced flexibility (e.g., due to materials, dimensions, etc.), with less shear force being imparted upon the support body 922 upon final implantation.

Each of the stimulation electrodes 924 are formed of an electrically conductive material appropriate for delivering stimulation energy within the human body. The stimulation electrodes 924 can assume any of the constructions of the present disclosure, and can, for example, be provided as elongated or rectangular block electrodes; in other embodiments, one or more of the stimulation electrodes 924 can have other shapes (e.g., square, cylinder, etc.) or constructions (e.g., akin to a wire, a coil, etc.). The stimulation electrodes 924 are arranged along the support body 922 so as to provide an exposed surface (from which stimulation energy is emitted) at or relative to the top face 944 of the support body 922. The stimulation electrodes 924 are encapsulated and electrically isolated from one another by the support body 922. In some embodiments, one or more of the stimulation electrodes 924 can be electrically common. Though not visible in the view of FIG. 13, individual, electrically isolated wire(s) can extend from each of the stimulation electrodes 924 within a thickness of the support body 922.

A collective arrangement of the stimulation electrodes 924 relative to the central minor axis I can be symmetric, and includes a first end electrode 924a and a second end electrode 924b. The first end electrode 924a is the stimulation electrode most proximate the first end 940 of the support body 922, and the second end electrode 924b is the stimulation electrode most proximate the second end 942 of the support body 922. Thus, the first end electrode 924a can be viewed as the leading electrode of the plurality of stimulation electrodes 924, and the second end electrode 924b can be viewed as the trailing electrode of the plurality of stimulation electrodes 924. Any number of stimulation electrodes 924 can be intermediately located between the first and second end electrodes 924a, 924b. Regardless of the number, shape, orientation, and configuration of the stimulation electrodes 924 provided with the simulation electrode assembly 920, a distance between the opposing, first and second end electrodes 924a, 924b serves as the effective length EL of the stimulation electrode assembly 920. Commensurate with the descriptions above, the stimulation electrode assembly 920 is configured such that the effective length EL corresponds with (e.g., approximates or exceeds) an expected span dimension of an anatomical target site upon final implant, for example the expected span dimension of a bilateral anatomical target site (e.g., and with additional reference to FIG. 4, the span distance S1 between the left and right hypoglossal nerves 130a, 130b in or near a region of corresponding innervation with the left and right genioglossus muscles 114a, 114b; the span distance S2 between the left and right hypoglossal nerves 130a, 130b in or near a region of corresponding innervation with the left and right hyoglossus muscles 118a, 118b; the span distance between nerve endings of the left and right hypoglossal nerves 130a, 130b; etc.).

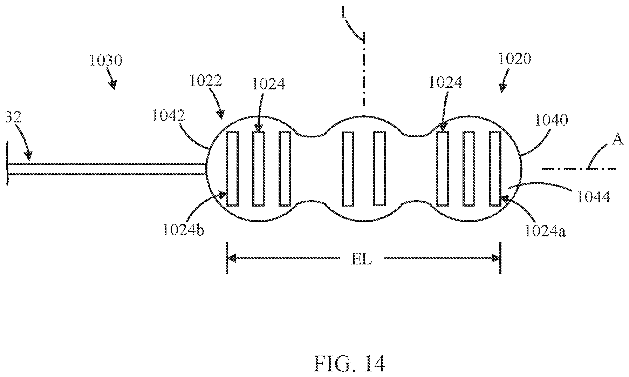

Another stimulation electrode assembly 1020 in accordance with principles of the present disclosure is shown in simplified form in FIG. 14. The stimulation electrode assembly 1020 includes a support body 1022 and a plurality or array of stimulation electrodes 1024. The stimulation electrode assembly 1020 is optionally provided as part of a stimulation lead 1030 that further includes the lead body 32 as described above.

The support body 1022 is akin to the support body 22 (FIG. 1) described above, and is configured to maintain the stimulation electrodes 1024 (as well as other optional electrical components) in an electrically isolated manner. The support body 1022 is formed of a biocompatible material appropriate for implantation into the human body. As described in greater detail below, the support body 1022 can form or carry one or more features that facilitate one or both of implantation and fixation. Regardless, a form factor or foot print or shape of the support body 1022 defines a first end 1040 opposite a second end 1042, and a top face 1044 opposite a bottom face (hidden in the view). With optional embodiments in which the lead body 32 is provided, the first end 1040 can be considered a leading end (e.g., as the first end 1040 is opposite the lead body 32) of the stimulation electrode assembly 1020, and the second end 1042 can be considered a trailing end. A shape of the support body 1022 defines a length between the first and second ends 1040, 1042, and a width perpendicular to the length. The length can be greater than the width, and a central major axis A of the shape of the support body 1022 is defined along or relative to the length. A central minor axis I of the support body 1022 is defined perpendicular to the central major axis A, mid-way between the first and second ends 1040, 1042.

With the non-limiting example of FIG. 14, the support body 1022 can have or define a variable width, for example described by a contoured perimeter shape. With these and related embodiments, the thinned regions of the support body 1022 can exhibit enhanced flexibility. Further, the contoured shape can promote tissue ingrowth along the support body 1022, thus facilitating passive fixation of the stimulation electrode assembly 1020 following implant. The contoured perimeter shape can be symmetrical relative to the major central axis A and relative to the minor central axis I as shown; in other embodiments, a non-uniform contoured perimeter shape can be employed.

Each of the stimulation electrodes 1024 are formed of an electrically conductive material appropriate for delivering stimulation energy within the human body. The stimulation electrodes 1024 can assume any of the constructions of the present disclosure, and can, for example, be provided as elongated or rectangular block electrodes; in other embodiments, one or more of the stimulation electrodes 1024 can have other shapes (e.g., square, cylinder, etc.) or constructions (e.g., akin to a wire, a coil, etc.). The stimulation electrodes 1024 are arranged along the support body 1022 so as to provide an exposed surface (from which stimulation energy is emitted) at or relative to the top face 1044 of the support body 1022. The stimulation electrodes 1024 are encapsulated and electrically isolated from one another by the support body 1022. In some embodiments, one or more of the stimulation electrodes 1024 can be electrically common. Though not visible in the view of FIG. 14, individual, electrically isolated wire(s) can extend from each of the stimulation electrodes 1024 within a thickness of the support body 1022.

A collective arrangement of the stimulation electrodes 1024 relative to the central minor axis I can be symmetric, and includes a first end electrode 1024a and a second end electrode 1024b. The first end electrode 1024a is the stimulation electrode most proximate the first end 1040 of the support body 1022, and the second end electrode 1024b is the stimulation electrode most proximate the second end 1042 of the support body 1022. Thus, the first end electrode 1024a can be viewed as the leading electrode of the plurality of stimulation electrodes 1024, and the second end electrode 1024b can be viewed as the trailing electrode of the plurality of stimulation electrodes 1024. Any number of stimulation electrodes 1024 can be intermediately located between the first and second end electrodes 1024a, 1024b. Regardless of the number, shape, orientation, and configuration of the stimulation electrodes 1024 provided with the simulation electrode assembly 1020, a distance between the opposing, first and second end electrodes 1024a, 1024b serves as the effective length EL of the stimulation electrode assembly 1020. Commensurate with the descriptions above, the stimulation electrode assembly 1020 is configured such that the effective length EL corresponds with (e.g., approximates or exceeds) an expected span dimension of an anatomical target site upon final implant, for example the expected span dimension of a bilateral anatomical target site (e.g., and with additional reference to FIG. 4, the span distance S1 between the left and right hypoglossal nerves 130a, 130b in or near a region of corresponding innervation with the left and right genioglossus muscles 114a, 114b; the span distance S2 between the left and right hypoglossal nerves 130a, 130b in or near a region of corresponding innervation with the left and right hyoglossus muscles 118a, 118b; the span distance between nerve endings of the left and right hypoglossal nerves 130a, 130b; etc.).

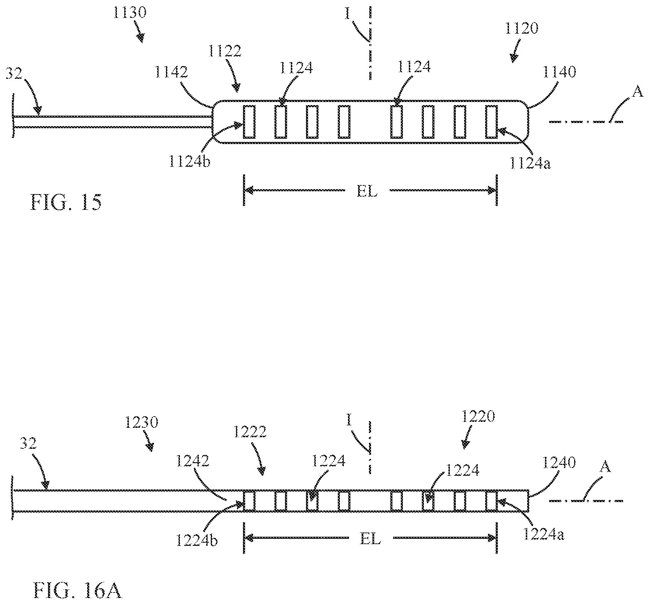

Another stimulation electrode assembly 1120 in accordance with principles of the present disclosure is shown in simplified form in FIG. 15. The stimulation electrode assembly 1120 includes a support body 1122 and a plurality or array of stimulation electrodes 1124. The stimulation electrode assembly 1120 is optionally provided as part of a stimulation lead 1130 that further includes the lead body 32 as described above.

The support body 1122 is akin to the support body 22 (FIG. 1) described above, and is configured to maintain the stimulation electrodes 1124 (as well as other optional electrical components) in an electrically isolated manner. The support body 1122 is formed of a biocompatible material appropriate for implantation into the human body. As described in greater detail below, the support body 1122 can form or carry one or more features that facilitate one or both of implantation and fixation. Regardless, a form factor or foot print or shape of the support body 1122 defines a first end 1140 opposite a second end 1142, and a top face (visible in the view) opposite a bottom face (hidden in the view). With optional embodiments in which the lead body 32 is provided, the first end 1140 can be considered a leading end (e.g., as the first end 1140 is opposite the lead body 32) of the stimulation electrode assembly 1120, and the second end 1142 can be considered a trailing end. A shape of the support body 1122 defines a length between the first and second ends 1140, 1142, and a width perpendicular to the length. The length can be greater than the width, and a central major axis A of the shape of the support body 1122 is defined along or relative to the length. A central minor axis I of the support body 1122 is defined perpendicular to the central major axis A, mid-way between the first and second ends 1140, 1142.

With the non-limiting example of FIG. 15, the support body 1122 can have or define a reduced or narrower width as compared to at least some of the other stimulation electrode assemblies of the present disclosure. For example, a width of the support body 1122 can approximate or be slightly greater than an outer dimension (e.g., diameter) of the lead body 32, such that the support body 1122 is akin to a ribbon.

Each of the stimulation electrodes 1124 are formed of an electrically conductive material appropriate for delivering stimulation energy within the human body. The stimulation electrodes 1124 can assume any of the constructions of the present disclosure, and can, for example, be provided as elongated or rectangular block electrodes; in other embodiments, one or more of the stimulation electrodes 1124 can have other shapes (e.g., square, cylinder, etc.) or constructions (e.g., akin to a wire, a coil, etc.). The stimulation electrodes 1124 are arranged along the support body 1122 so as to provide an exposed surface (from which stimulation energy is emitted) at or relative to the top face of the support body 1122. The stimulation electrodes 1124 are encapsulated and electrically isolated from one another by the support body 1122. In some embodiments, one or more of the stimulation electrodes 1124 can be electrically common. Though not visible in the view of FIG. 15, individual, electrically isolated wire(s) can extend from each of the stimulation electrodes 1124 within a thickness of the support body 1122.

A collective arrangement of the stimulation electrodes 1124 relative to the central minor axis I can be symmetric, and includes a first end electrode 1124a and a second end electrode 1124b. The first end electrode 1124a is the stimulation electrode most proximate the first end 1140 of the support body 1122, and the second end electrode 1124b is the stimulation electrode most proximate the second end 1142 of the support body 1122. Thus, the first end electrode 1124a can be viewed as the leading electrode of the plurality of stimulation electrodes 1124, and the second end electrode 1124b can be viewed as the trailing electrode of the plurality of stimulation electrodes 1124. Any number of stimulation electrodes 1124 can be intermediately located between the first and second end electrodes 1124a, 1124b. Regardless of the number, shape, orientation, and configuration of the stimulation electrodes 1124 provided with the simulation electrode assembly 1120, a distance between the opposing, first and second end electrodes 1124a, 1124b serves as the effective length EL of the stimulation electrode assembly 1120. Commensurate with the descriptions above, the stimulation electrode assembly 1120 is configured such that the effective length **EL** corresponds with (e.g., approximates or exceeds) an expected span dimension of an anatomical target site upon final implant, for example the expected span dimension of a bilateral anatomical target site (e.g., and with additional reference to FIG. 4, the span distance S1 between the left and right hypoglossal nerves 130a, 130b in or near a region of corresponding innervation with the left and right genioglossus muscles 114a, 114b; the span distance S2 between the left and right hypoglossal nerves 130a, 130b in or near a region of corresponding innervation with the left and right hyoglossus muscles 118a, 118b; the span distance between nerve endings of the left and right hypoglossal nerves 130a, 130b; etc.).

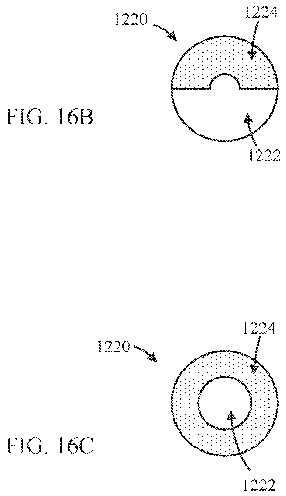

Another, related embodiment stimulation electrode assembly 1220 in accordance with principles of the present disclosure is shown in simplified form in FIGS. 16A and 16B. The stimulation electrode assembly 1220 includes a support body 1222 and a plurality or array of stimulation electrodes 1224. The stimulation electrode assembly 1220 is optionally provided as part of a stimulation lead 1230 that further includes the lead body 32 as described above.

The support body 1222 is akin to the support body 22 (FIG. 1) described above, and is configured to maintain the stimulation electrodes 1224 (as well as other optional electrical components) in an electrically isolated manner. The support body 1222 is formed of a biocompatible material appropriate for implantation into the human body. As described in greater detail below, the support body 1222 can form or carry one or more features that facilitate one or both of implantation and fixation. Regardless, a form factor or foot print or shape of the support body 1222 defines a first end 1240 opposite a second end 1242, and a top face (visible in the view of FIG. 16A) opposite a bottom face (hidden in the view). With optional embodiments in which the lead body 32 is provided, the first end 1240 can be considered a leading end (e.g., as the first end 1240 is opposite the lead body 32) of the stimulation electrode assembly 1220, and the second end 1242 can be considered a trailing end. A shape of the support body 1222 defines a length between the first and second ends 1240, 1242, and a minor outer dimension (e.g., diameter or width) perpendicular to the length. The length can be greater than the minor outer dimension, and a central major axis A of the shape of the support body 1222 is defined along or relative to the length. A central minor axis I of the support body 1222 is defined perpendicular to the central major axis A, mid-way between the first and second ends 1240, 1242.

As best reflected by FIG. 16A, the support body 1222 can have or define a reduced or narrower width as compared to at least some of the other stimulation electrode assemblies of the present disclosure. For example, an outer minor dimension of the support body 1222 can approximate that of the lead body 32, such that the support body 1222 is akin to a ribbon or a continuation of the lead body 32.

Each of the stimulation electrodes 1224 are formed of an electrically conductive material appropriate for delivering stimulation energy within the human body. The stimulation electrodes 1224 can assume any of the constructions of the present disclosure. The stimulation electrodes 1224 are arranged along the support body 1222 so as to provide an exposed surface (from which stimulation energy is emitted) at or relative to the top face of the support body 1222. The stimulation electrodes 1224 are encapsulated and electrically isolated from one another by the support body 1222. In some embodiments, and as best reflected by FIG. 16B, one or more or all of the stimulation electrodes 1224 can be, or can be akin to, a split ring electrode. In some embodiments, one or more of the stimulation electrodes 1224 can be electrically common. Though not visible in the views, individual, electrically isolated wire(s) can extend from each of the stimulation electrodes 1224 within a thickness of the support body 1222.

In other embodiments, one or more or all of the stimulation electrodes 1224 can have an axially symmetric shape. For example, FIG. 16C illustrates an alternative stimulation electrode 1224' useful with the stimulation electrode assembly 1220. The stimulation electrode 1224' extends along an entire circumference of the support body 1222, akin to conventional stimulation lead electrodes. Returning to FIG. 16A, some or all of the stimulation electrodes 1224 can each have a split ring shape (as in FIG. 16B) and some or all of the stimulation electrodes 1224 can each be fully circumferential (as in FIG. 16C and akin to a traditional stimulation lead). Thus, some stimulation electrode assemblies and corresponding stimulation leads of the present disclosure can be akin to a traditional axial symmetric stimulation lead.

A collective arrangement of the stimulation electrodes 1224 relative to the central minor axis I can be symmetric, and includes a first end electrode 1224a and a second end electrode 1224b. The first end electrode 1224a is the stimulation electrode most proximate the first end 1240 of the support body 1222, and the second end electrode 1224b is the stimulation electrode most proximate the second end 1242 of the support body 1222. Thus, the first end electrode 1224a can be viewed as the leading electrode of the plurality of stimulation electrodes 1224, and the second end electrode 1224b can be viewed as the trailing electrode of the plurality of stimulation electrodes 1224. Any number of stimulation electrodes 1224 can be intermediately located between the first and second end electrodes 1224a, 1224b. Regardless of the number, shape, orientation, and configuration of the stimulation electrodes 1224 provided with the simulation electrode assembly 1220, a distance between the opposing, first and second end electrodes 1224a, 1224b serves as the effective length EL of the stimulation electrode assembly 1220. Commensurate with the descriptions above, the stimulation electrode assembly 1220 is configured such that the effective length EL corresponds with (e.g., approximates or exceeds) an expected span dimension of an anatomical target site upon final implant, for example the expected span dimension of a bilateral anatomical target site (e.g., and with additional reference to FIG. 4, the span distance S1 between the left and right hypoglossal nerves 130a, 130b in or near a region of corresponding innervation with the left and right genioglossus muscles 114a, 114b; the span distance S2 between the left and right hypoglossal nerves 130a, 130b in or near a region of corresponding innervation with the left and right hyoglossus muscles 118a, 118b; the span distance between nerve endings of the left and right hypoglossal nerves 130a, 130b; etc.).

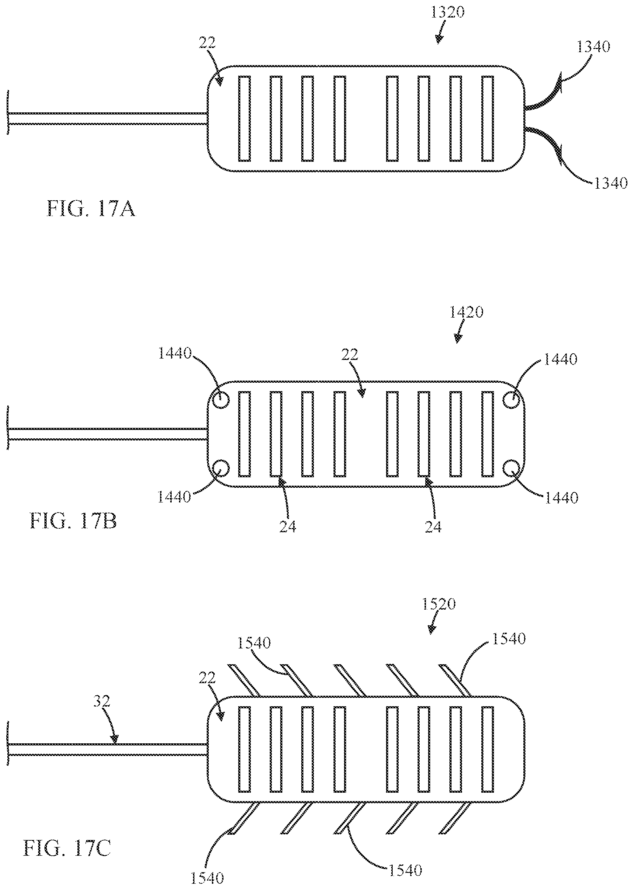

With any of the stimulation electrode assemblies of the present disclosure, features can be included or provided that promote fixation during or following implant. The provided fixation can be activate fixation. For example, FIG. 17A illustrates another stimulation electrode assembly 1320 that is highly akin to the stimulation electrode assembly 20 (FIGS. 1A and 1B), and further includes one or more extendable/retractable tines 1340 (e.g., similar to metal tines used in micro-pacemakers) carried by the support body 22. Other active fixation device formats akin to a tine (e.g., helix) can be utilized. A mechanism for activating or deploying the active fixation device is carried by the support body 22 and is connected to an actuator that can be accessed by a clinician external the patient during an implantation procedure (e.g., a wire extending from the support body 22). With these and related embodiments, the active fixation device(s) can be in a retracted state or position (e.g., withdrawn within the support body 22) during initial implantation of the simulation electrode device 1320 (with the support body 22 providing a low profile conducive to implant). Upon locating the stimulation electrode assembly 1320 at a desired target site, the active fixation device(s) is caused to deploy relative to the support body 22, providing fixation into tissue surrounding the target site.

Alternatively or in addition, the stimulation electrode assemblies of the present disclosure can incorporate feature(s) that provide passive fixation. For example, a mesh material configured to promote tissue ingrowth (e.g., akin to a hernia mesh) can be formed over or incorporated into the support body 22. Other texturing can be provided on a surface to interact with/promote tissue ingrowth, for example a texturing akin to Velcro^{®}-style loops. Similarly, and as shown with respect to the stimulation electrode assembly 1420 of FIG. 17B (that is otherwise highly akin to the stimulation electrode assembly 20 (FIGS. 1A and 1B)), one or more through holes 1440 can be formed through the support body 22 for fibrotic tissue to grow. One or more of the through holes 1440 can alternatively serve to receive a suture that in turn provides positive fixation at the implant site. A construction of one or more of the stimulation electrodes 24 can be selected to promote tissue growth (e.g., the coil electrodes described above). One or more dissolvable suture barbs can be carried by the support body 22. Similarly, and as shown with respect to the stimulation electrode assembly 1520 of FIG. 17C (that is otherwise highly akin to the stimulation electrode assembly 20 (FIGS. 1A and 1B)), one or more passive tines 1540 (e.g., made from a material such as silicone, polyurethane or the like) can be carried by the support body 22. A mechanical protraction can be provided along the lead body 32, sized, shaped, and located relative to the stimulation electrode assembly 1520 so as to not "fit" into the either the tunnel created at the target site (at which the stimulation electrode assembly 1520 is position upon final implant) nor the tunnel created to the location of the stimulation device (e.g., IPG) that the lead body 32 is subsequently connected. Similarly, the exterior perimeter of the support body 22 can be contoured or implement protrusions such that fibrotic encapsulation would occur around the perimeter (e.g., the non-limiting example of FIG. 14). With these and similar embodiments, the mechanical protrusion would effectively act to "anchor" the lead body 32, and thus the stimulation electrode assembly 20, in both directions.

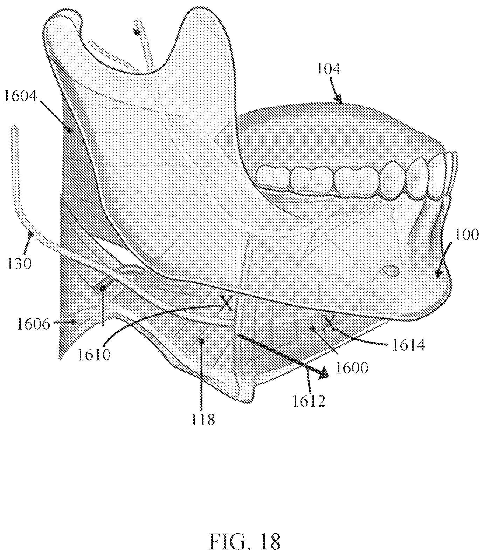

Any of the stimulation electrode assemblies of the present disclosure (e.g., the stimulation electrode assemblies of FIGS. 1 and 6-17C and variations thereof) can be implanted to a bilateral target site, such as the bilateral target site 200 of FIG. 5A intended to facilitate selective simulation of the left and right hypoglossal nerves 130a, 130b (e.g., the third medial nerve trunk second branch 150a of the left hypoglossal nerve 130a and the third medial nerve trunk second branch 150b of the right hypoglossal nerve 130b; nerve endings associated with the left hypoglossal nerve 130a and the right hypoglossal nerve 130b) in various manners. In some embodiments, methods of the present disclosure provide for delivery of a bilateral stimulation electrode assembly in a vicinity of the left and right hypoglossal nerves 130a, 130b via a small incision at one side of the patient's chin, and tunneling across the mid-line M of the chin to the target site (e.g., an incision is made at the left side of the patient's chin, and a tunnel is formed from the left side toward the patient's right side, crossing the mid-line M; or vice-versa). As a point of reference, FIG. 18 illustrates anatomy of the symphysis of mandible 100, the tongue 104 and the hypoglossal nerve 130. The hyoglossus muscle 118 extends to the tongue 104 as described above. In addition, FIG. 18 shows a mylohyoid muscle 1600 that runs from the symphysis of mandible 100 (referenced generally) to the hyoid bone, a superior pharyngeal constrictor muscle 1604, and a middle pharyngeal constrictor muscle 1606.

With the above anatomy in mind, one example method of the present disclosure appropriate for achieving implantation of the stimulation electrode assembly at the target site 200 of FIG. 5A can include forming an incision through the patient's skin at a location generally indicated at 1610 in FIG. 18. With cross-reference between FIGS. 5A and 18, the incision is sufficient to access the mylohyoid muscle 1600. The clinician can then retract the mylohyoid muscle 1600 in a direction of arrow 1612, access tissue behind the mylohyoid muscle 1600, and create a tunnel to the target site 200 (FIG. 5A), including the tunnel crossing the mid-line M. In other embodiments, a puncture is formed at a location generally indicated at 1614, through the patient's skin and the mylohyoid muscle 1600. In related embodiments, the puncture could be formed at approximately the location 1614 (i.e., at either the left side or the right side of the patient) through the hyoglossus muscle 118 or the genioglossus muscle 114. A surgical punch (not shown) can be employed, for example a tool with a predefined blade width on the distal end; instead of cutting like a scalpel, the tool is pushed against the patient's skin to make an incision with a defined width (e.g. commensurate with a width of the stimulation electrode assembly to be implanted). Alternatively, traditional surgical tools (e.g., scalpel, cautery, etc.) could be utilized to make the incision. A tunnel is then formed through the puncture, extending across the mid-line M. With these and similar techniques, the tunnel can be formed to locate the target site 200 "under" the hyoglossus muscle 118 (e.g., one or both of the left and right hyoglossus muscles 118a, 118b) and the genioglossus muscle 114 (e.g., one or both of the left and right genioglossus muscles 114a, 114b). The target location for the tunnel is adjacent to the hypoglossal nerve on the left and right side of the anatomy. In other embodiments, the target location for the tunnel is into the base of the genioglossus muscle. As is known in the art, additional tunneling can be performed through the incision or puncture, for example a tunnel for placement of the lead body 32 (FIG. 1).

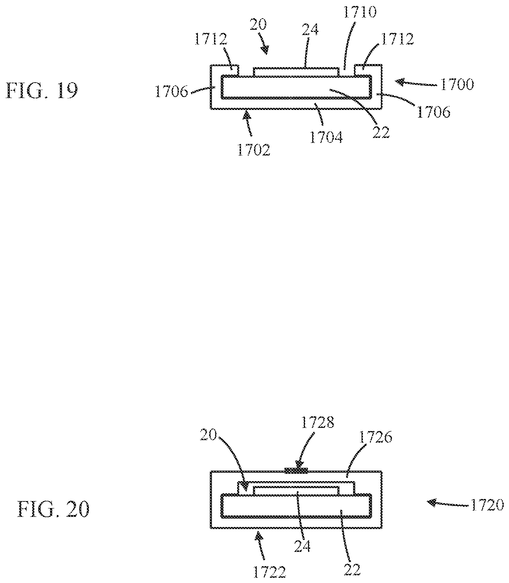

Regardless of how the tunnel to the target site 200 is created, the stimulation electrode assembly is then delivered to the target site 200 via the tunnel. Some embodiments of the present disclosure relate to tools for introducing or delivering the stimulation electrode assembly. One example of an introducer 1700 of the present disclosure is shown in simplified form in FIG. 19, along with the stimulation electrode assembly 20. It will be understood that the introducer 1700 can be used with any of the stimulation electrode assemblies of the present disclosure, and is not limited to the stimulation electrode assembly 20. The introducer 1700 includes a head 1702 carried at a distal end of a shaft (not shown). The head 1702 includes a base 1704 and side walls 1706 that combine to define a channel or pocket sized and shaped to receive the support body 22. A gap or spacing 1710 is defined between the side walls 1706 opposite the base 1704. The gap 1710 is sized and shape in accordance with a size and shape of the stimulation electrodes 24 (one of which is visible in the view of FIG. 19). For example, a lip 1712 can extend inwardly from each of the side walls 1706 that serve to partially close the channel (e.g., the support body 22 is captured between the base 1704 and the lips 1712), with the gap 1710 defined between the lips 1712. Regardless, a side of the head 1702 opposite the base 1704 is sufficiently open to expose the stimulation electrodes 24. During a delivery procedure, the stimulation electrode assembly 20 is loaded to the introducer 1700 as shown, and the introducer 1700 is manipulated to advance the stimulation electrode assembly 20 to the target site within the patient (e.g., the stimulation electrode assembly 20 is advanced into the patient from one side of the chin and progressed through a previously-created tunnel to the target site extending across a mid-line of the patient's chin). With the stimulation electrode assembly 20 still retained by the introducer 1700, electrical testing can be performed to verify or confirm a location of the various stimulation electrodes 24 relative to anatomy of interest (e.g., the left and right hypoglossal nerves), for example by selectively delivering stimulation energy to one or more or all of the stimulation electrodes 24 that in turn deliver or apply the energy to the patient through the gap 1710. Once the clinician is satisfied with a location of the stimulation electrode assembly 20 within the patient, the introducer 1700 can be removed.

Another example of an introducer 1720 of the present disclosure is shown in simplified form in FIG. 20, along with the stimulation electrode assembly 20. It will be understood that the introducer 1720 can be used with any of the stimulation electrode assemblies of the present disclosure, and is not limited to the stimulation electrode assembly 20. The introducer 1720 includes a head 1722 carried at a distal end of a shaft (not shown). The head 1722 defines a closed channel or pocket sized and shaped to receive the stimulation electrode assembly 20. Unlike the introducer 1700 of FIG. 19, the head 1722 include an upper wall 1726 that extends over the stimulation electrode assembly 20 upon loading of the stimulation electrode assembly 20 into the channel. In some embodiments, the introducer 1720 can further include one or more test electrodes 1728 carried by, or embedded into a thickness of, the upper wall 1726, along with wiring (not shown) extending from the corresponding test electrode 1728 along the shaft. In some embodiments, the number of test electrodes 1728 provided with the introducer 1720 can correspond with the number of stimulation electrodes 24 provided with the stimulation electrode assembly 20, and the test electrodes 1728 are arranged on or along the upper wall 1726 in accordance with the pattern of the stimulation electrodes 24 carried by the support body 22. The introducer 1720 can further include alignment features (e.g., protrusion, etc.) that promote positioning of the stimulation electrode assembly 20 within the channel such that in a final, loaded state of the stimulation electrode assembly 20, each of the stimulation electrodes 24 is aligned with a corresponding one of the test electrodes 1728. In other embodiments, the number of the test electrodes 1728 provided with the introducer 1720 is less than the number of stimulation electrodes 24, including only a single one of the test electrodes 1728.

During a delivery procedure, the stimulation electrode assembly 22 is loaded to the introducer 1720 as shown, and the introducer 1720 is manipulated to advance the stimulation electrode assembly 20 to the target site within the patient (e.g., the stimulation electrode assembly 20 is advanced into the patient from one side of the chin and progressed through a previously-created tunnel to the target site extending across a mid-line of the patient's chin). With the stimulation electrode assembly 20 still retained by the introducer 1720, electrical testing can be performed to verify or confirm a location of the various stimulation electrodes 24 relative to anatomy of interest (e.g., the left and right hypoglossal nerves), for example by selectively delivering stimulation energy to one or more or all of the test electrodes 1728 that in turn deliver or apply the energy to the patient. Once the clinician is satisfied with a location of the stimulation electrode assembly 20 within the patient, the introducer 1720 can be removed. In some embodiments, the introducer 1720 can be, or can be akin to, a peel away introducer or sheath, that facilitates disassembly from the stimulation electrode assembly 20 (e.g., perforations or slit lines can be formed through a thickness of the head 1722). In other embodiments, the introducer 1720 can first be inserted into the patient prior to final loading of the stimulation electrode assembly 20, and the head 1722 directed to the target site. Testing can be done using the test electrode(s) 1728. Once the clinician is satisfied with a location of the head 1722 relative to the target site, the stimulation electrode assembly 20 can then be delivered through the introducer 1720 to a location within the head 1722.

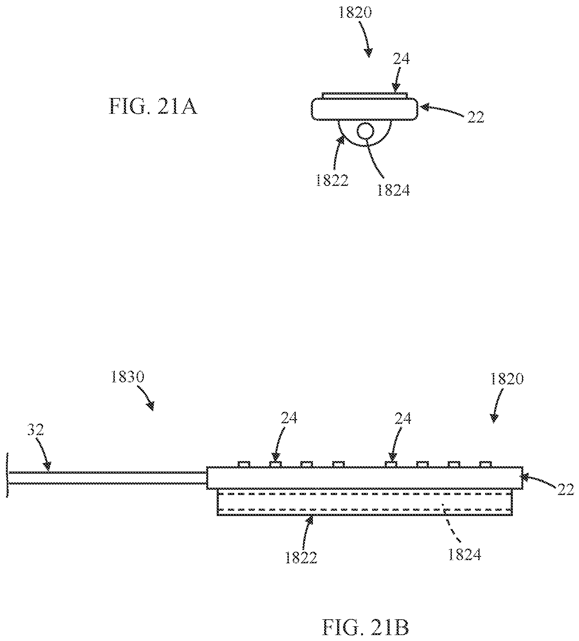

In some embodiments, the stimulation electrode assemblies of the present disclosure can include or incorporate one or more features that facilitate delivery to a target site. For example, another stimulation electrode assembly 1820 in accordance with principles of the present disclosure is shown in simplified form in FIGS. 21A and 21B. The stimulation electrode assembly 1820 can be akin to any of the stimulation electrode assemblies described above, and can include, for example, the support body 22 and the stimulation electrodes 24 as described above (or the support body and stimulation electrode arrangement of any other embodiment of the present disclosure). In addition, the stimulation electrode assembly 1820 includes a guide body 1822 attached to or integrally formed with the support body 22. The guide body 1822 defines a lumen 1824 sized to slidably receive a delivery tool or device appropriate for guiding the stimulation electrode assembly 1820 to a target site, such as a guidewire, catheter, stylet, etc. The stimulation electrode assembly 1820 is optionally provided as part of a stimulation lead 1830 that further includes the lead body 32 as described above.

While the lumen 1824 is shown as extending along a length approximating a length of the support body 22, other geometries or configurations are also acceptable. For example, in other embodiments, a length of the guide body 1822, and thus a length of the lumen 1824, can be substantively less than a length of the support body 22 (e.g., no greater than approximately 50% of a length of the support body 22). Alternatively, the stimulation lead 1830 can be configured or constructed such that the lumen 1824 extends from the support body 22 along (e.g., within) at least a portion of the lead body 32, for example an entire length of the lead body 32. Regardless, with some non-limiting methods of the present disclosure, a distal region of a guide device (e.g., guide wire, catheter, or stylet) is initially advanced to the target site. The guide device is slidably disposed within the lumen 1824. The stimulation electrode assembly 1820 is then advanced over the guide device to the target site. Once the clinician is satisfied with a location of the stimulation electrode assembly 1820 relative to the target site (e.g., following testing), the guide device can be retracted from the lumen and the patient. Alternatively, if the lumen terminates near the proximal end of the support body 22 but does not pass through an entirety of the support body 22, a stylet can be used to effectively push the support body 22 into a previously-made delivery channel.

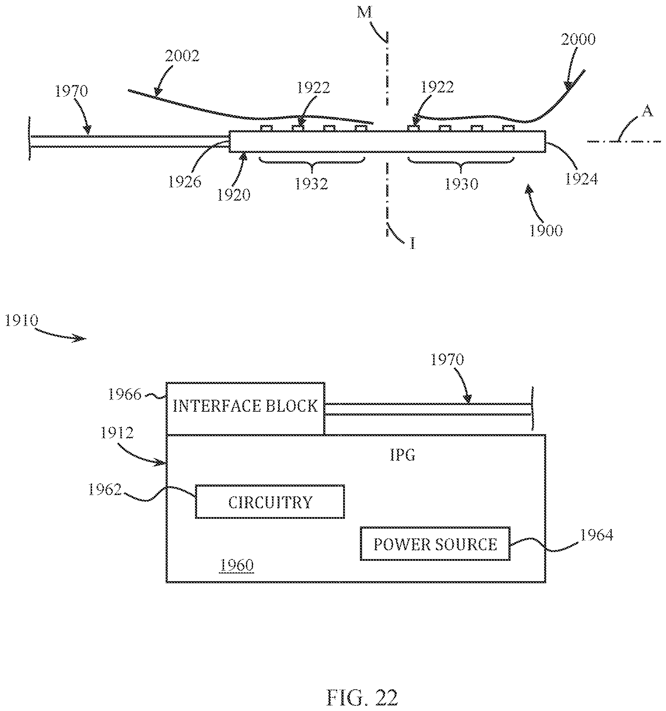

Regardless of the method of delivery and implantation, the stimulation electrode assemblies of the present disclosure can be operated (e.g., supplied with electrical stimulation energy, for example via an implantable pulse generator (IPG)) to deliver stimulation therapy to the patient in various manners, for example on a bilateral basis. As a point of reference, FIG. 22 is a simplified representation of a final implant location of a stimulation electrode assembly 1900 relative to a first nerve 2000 and a second nerve 2002 of the patient. With the non-limiting example of FIG. 22, the simulation electrode assembly 1900 is provided as part of a stimulation system 1910 that further includes an implantable pulse generator (IPG) assembly 1912 as described in greater detail below. The stimulation electrode assembly 1900 can have any of the constructions of the present disclosure, and generally includes a support body 1920 carrying a plurality of stimulation electrodes 1922. Commensurate with the descriptions above, the support body 1920 has an elongated shape, defining a length greater than a width. A central major axis A is defined by the support body 1920 in a direction of the length. A central minor axis I is defined perpendicular to the central major axis A mid-way between opposing ends 1924, 1926 of the support body 1920. The stimulation electrodes 1922 can have any of the constructions, shapes, patterns, etc., of the present disclosure. In more general terms, the plurality of stimulation electrodes 1922 can be viewed as providing a first sub-group of electrodes 1930 at one side of the central minor axis I (e.g., between the central minor axis I and the first end 1924) and a second sub-group of electrodes 1932 at an opposite side of the central minor axis I (e.g., between the central minor axis I and the second end 1926). A configuration of the first sub-group of electrodes 1930 can be identical to that of the second sub-group of electrodes 1932 (i.e., the first and second sub-groups of electrodes 1930, 1932 are symmetric relative to the central minor axis I); in other embodiments, the first and second sub-groups of electrodes 1930, 1932 can differ from one another in terms of one or more of number of stimulation electrodes, type of stimulation electrodes, shape of stimulation electrodes, location of stimulation electrodes, etc. While FIG. 22 reflects the first and second sub-groups of electrodes 1930, 1932 as each including four of the stimulation electrodes 1922, any other number, either greater or lesser (including a single stimulation electrode 1922) is equally acceptable.

The IPG assembly 1912 can include a housing 1960 containing circuitry 1962 and a power source 1964 (e.g., battery), and an interface block or header-connector 1966 carried or formed by the housing 1960. The housing 1960 is configured to render the IPG assembly 1912 appropriate for implantation into a human body, and can incorporate biocompatible materials and hermetic seal(s). The circuitry 1962 can include circuitry components and wiring apparent to one of ordinary skill appropriate for generating desired stimulation signals (e.g., converting energy provided by the power source 1964 into a desired stimulation signal), for example in the form of a stimulation engine. In some embodiments, the circuitry 1962 can include telemetry components for communication with external devices as is known in the art. The interface block 1966 is configured to facilitate coupling of the IPG assembly 1916 with the stimulation electrode assembly 1900, for example via a lead body 1970 coupled to the stimulation electrode assembly 1900 as described above. Upon generation via the circuitry 1962 (for example, as controlled or prompted by algorithms or a therapy manager programmed to or operated by the circuitry 1962), a stimulation signal is selectively transmitted to the interface block 1966 for delivery to the selected ones of the stimulation electrodes 1922. In other embodiments, the stimulation electrode assembly 1900 can be more directly connected to or carried by the power source (e.g., such as with a microstimulator configuration).

In some embodiments, the nerves 2000, 2002 are a bilateral nerve pair extending from opposite sides of the patient's mid-line M, such as the left and right hypoglossal nerves. In some embodiments, the first nerve 2000 can be the third medial nerve trunk second branch of the left hypoglossal nerve, and the second nerve 2002 can be the third medial nerve trunk second branch of the right hypoglossal nerve. A number of other nerves or nerve segments (including, but not limited to, nerve endings or the ends of nerve fibers) can be implicated by the devices, systems and methods of the present disclosure. Regardless, the stimulation electrode assembly 1900 is located, upon final implant, such that the first sub-group of electrodes 1930 is proximate or positioned to affect the first nerve 2000, and the second sub-group of electrodes 1932 is proximate or positioned to affect the second nerve 2002, for example by the stimulation electrode assembly 1900 extending across the mid-line M.

As schematically reflected by FIG. 22, upon final implant, the stimulation electrodes 1922 of the first sub-group of electrodes 1930 need not be in direct, physical contact with the first nerve 2000 (although one or more of the stimulation electrodes 1922 of the first sub-group of electrodes 1930 may be in direct, physical contact with the first nerve 2000). Similarly, the stimulation electrodes 1922 of the second sub-group of electrodes 1932 need not be in direct, physical contact with the second nerve 2002 (although one or more of the stimulation electrodes 1922 of the second sub-group of electrodes 1932 may be in direct, physical contact with the second nerve 2002). With some methods of the present disclosure, the stimulation electrode assembly 1900 is located or implanted in muscle and/or fatty tissue of the patient in close proximity to, but not in physical contact with, the nerves 2000, 2002. A distance between respective ones of the stimulation electrodes 1922 and the corresponding targeted nerve 2000, 2002 can vary (e.g., a distance between individual ones of the stimulation electrodes 1922 of the first sub-group of electrodes 1930 and the first nerve 2000 upon final implant may or may not be identical).

The stimulation electrode assembly 1900 can be operated as part of the stimulation system 1910 (that further includes an energy source (e.g., the IPG assembly 1912) electrically connected to the stimulation electrodes 1922) to deliver stimulation energy to one or both of the nerves 2000, 2002 in various manners, for example via programming or algorithms provided with or operated upon the energy source in accordance with the present disclosure. In some examples, the stimulation is delivered to a nerve to cause a response in a corresponding innervated muscle. In some examples, the nerve (e.g., the nerves 2000, 2002) may be related to restoring upper airway patency, such as used in a method of treating sleep disordered breathing. In some embodiments, the stimulation electrode assembly 1900 is operated to deliver stimulation energy (via one or more of the stimulation electrodes 1922) at levels sufficiently high enough to stimulate a nerve, but sufficiently low enough to not overtly directly activate muscle tissue ("non-muscle stimulating nerve signals"). For example, with embodiments in which the stimulation electrodes 1922 are not in direct physical contact with the corresponding, most-proximate nerve 2000, 2002, the stimulation electrode assembly 1900 can be operated to essentially "spray" an electrical signal in a direction of the corresponding nerve 2000, 2002 at levels sufficient to stimulate the nerve 2000, 2002 but not overtly stimulate a muscle or other tissue in a path of the sprayed electrical signal.

In some embodiments, the methods of the present disclosure include providing stimulation energy to only one of the first sub-group of electrodes 1930 and the second sub-group of electrodes 1932. In other embodiments, the methods of the present disclosure include selectively providing stimulation energy to one or more of the stimulation electrodes 1922 of the first sub-group of electrodes 1930 and one or more of the stimulation electrodes 1922 of the second sub-group of electrodes 1932. With these and related embodiments, the stimulation electrode assembly 1900 can be operated to provide synchronous stimulation at, or from, the first and second sub-groups of electrodes 1930, 1932. For example, an interleaving pulse train can be delivered to the stimulation electrodes 1922 of the first and second sub-groups of electrodes 1930, 1932. Regardless, the synchronous stimulation of the nerves 2000, 2002 (e.g., bilateral stimulation) can be performed at energy level thresholds that are less than the threshold employed for unilateral nerve stimulation in treating the same disorder (e.g., SDB). The stimulation at the first and second sub-groups of electrodes 1930. 1932 may be interloped and not perfectly synched.

The particular format of the energy delivered to, and thus emitted from, the stimulation electrodes 1922 of the first sub-group 1930 can differ from that delivered to the stimulation electrodes 1922 of the second sub-group 1932. Moreover, the energy format provided to the stimulation electrodes 1922 within one or both of the sub-groups 1930, 1932 can differ. For example, the level of energy provided to individual ones of the stimulation electrodes 1922 within each of the sub-groups 1930, 1932 can vary based upon a distance between the individual electrode 1922 and the corresponding nerve 2000, 2002. Positive or negative energy can be delivered to selective ones of the stimulation electrodes 1922 within each of the sub-groups 1930, 1932. Selective ones of the stimulation electrodes 1922 of one or both of the first and second sub-groups 1930, 1932 can be operated to hyperpolarize a particular nerve segment. For example, upon final implant, a first one of the stimulation electrodes 1922 may be located proximate a segment of the nerve 2000, 2002 the stimulation of which causes a desired reaction in a first muscle and a second one of the stimulation electrodes 1922 is located proximate a segment of the nerve 2000, 2002 the stimulation of which causes an undesired reaction of a second muscle (e.g., the first muscle can be a tongue protrusor muscle and the second muscle can be a tongue retractor muscle); with these and related scenarios, the methods of the present disclosure can include simultaneously providing energy to the first stimulation electrode 1922 appropriate to prompt stimulation of the first nerve segment and activation of the corresponding first muscle, and energy to the second stimulation electrode 1922 appropriate to "stun" the second nerve second and limit or prevent activation of the corresponding second muscle. A voltage source or a current source can be utilized with the IPG assembly 1912. In some examples, one or more of the stimulation electrodes 1922 can be operated to provide unipolar stimulation to a selected nerve branch.

As further shown in FIG. 22, the lead body 1970 can be for chronic subcutaneous implantation (e.g. via tunneling) and to extend to a position adjacent a nerve (e.g. hypoglossal nerve and/or phrenic nerve). The stimulation electrode assembly 1900 may comprise the stimulation electrodes 1922 to interface with one or more nerves for stimulating the nerve(s) to treat a physiologic condition, such as sleep disordered breathing like obstructive sleep apnea, central sleep apnea, multiple-type sleep apneas, etc. The IPG assembly 1912 or similar device may comprise circuitry, power element, etc., to support control and operation of both a sensor and the stimulation electrodes 1922 (via the lead body 1970). In some examples, such control, operation, etc. may be implemented, at least in part, via a control portion (and related functions, portions, elements, engines, parameters, etc.).

With regard to the various examples of the present disclosure, in some examples, delivering stimulation to an upper airway patency nerve (e.g. a hypoglossal nerve) via the stimulation electrode(s) 1922 is to cause contraction of upper airway patency-related muscles, which may cause or maintain opening of the upper airway to prevent and/or treat obstructive sleep apnea. Similarly, such electrical stimulation may be applied to a phrenic nerve via the stimulation electrode(s) 1922 to cause contraction of the diaphragm as part of preventing or treating at least central sleep apnea. It will be further understood that some example methods may comprise treating both obstructive sleep apnea and central sleep apnea, such as but not limited to, instances of multiple-type sleep apnea in which both types of sleep apnea may be present at least some of the time. In some such instances, separate stimulation leads may be provided or a single stimulation lead may be provided but with a bifurcated distal portion with each separate distal portion extending to a respective one of the hypoglossal nerve and the phrenic nerve.

In some such examples, the contraction of the hypoglossal nerve and/or contraction of the phrenic nerve caused by electrical stimulation comprises a suprathreshold stimulation, which is in contrast to a subthreshold stimulation (e.g. mere tone) of such muscles. In one aspect, a suprathreshold intensity level corresponds to a stimulation energy greater than the nerve excitation threshold, such that the suprathreshold stimulation may provide for higher degrees (e.g. maximum, other) upper-airway clearance (i.e. patency) and sleep apnea therapy efficacy.

In some examples, a target intensity level of stimulation energy is selected, determined, implemented, etc. without regard to intentionally establishing a discomfort threshold of the patient (such as in response to such stimulation). Stated differently, in at least some examples, a target intensity level of stimulation may be implemented to provide the desired efficacious therapeutic effect in reducing sleep disordered breathing (SDB) without attempting to adjust or increase the target intensity level according to (or relative to) a discomfort threshold.

In some examples, the treatment period (during which stimulation may be applied at least part of the time) may comprise a period of time beginning with the patient turning on the therapy device and ending with the patient turning off the device. In some examples, the treatment period may comprise a selectable, predetermined start time (e.g. 10 p.m.) and selectable, predetermined stop time (e.g. 6 a.m.). In some examples, the treatment period may comprise a period of time between an auto-detected initiation of sleep and auto-detected awake-from-sleep time. With this in mind, the treatment period corresponds to a period during which a patient is sleeping such that the stimulation of the upper airway patency-related nerve and/or central sleep apnea-related nerve is generally not perceived by the patient and so that the stimulation coincides with the patient behavior (e.g. sleeping) during which the sleep disordered breathing behavior (e.g. central or obstructive sleep apnea) would be expected to occur.

In some examples the initiation or termination of the treatment period may be implemented automatically based on sensed sleep state information, which in turn may comprise sleep stage information.

To avoid enabling stimulation prior to the patient falling asleep, in some examples stimulation can be enabled after expiration of a timer started by the patient (to enable therapy with a remote control), or enabled automatically via sleep stage detection. To avoid continuing stimulation after the patient wakes, stimulation can be disabled by the patient using a remote control, or automatically via sleep stage detection. Accordingly, in at least some examples, these periods may be considered to be outside of the treatment period or may be considered as a startup portion and wind down portion, respectively, of a treatment period.

In some examples, stimulation of an upper airway patency-related nerve may be performed via open loop stimulation. In some examples, the open loop stimulation may refer to performing stimulation without use of any sensory feedback of any kind relative to the stimulation.

In some examples, the open loop stimulation may refer to stimulation performed without use of sensory feedback by which timing of the stimulation (e.g. synchronization) would otherwise be determined relative to respiratory information (e.g. respiratory cycles). However, in some such examples, some sensory feedback may be utilized to determine, in general, whether the patient should receive stimulation based on a severity of sleep apnea behavior.

Conversely, in some examples and as previously described in relation to at least several examples, stimulation of an upper airway patency-related nerve may be performed via closed loop stimulation. In some examples, the closed loop stimulation may refer to performing stimulation at least partially based on sensory feedback regarding parameters of the stimulation and/or effects of the stimulation.

In some examples, the closed loop stimulation may refer to stimulation performed via use of sensory feedback by which timing of the stimulation (e.g. synchronization) is determined relative to respiratory information, such as but not limited to respiratory cycle information, which may comprise onset, offset, duration, magnitude, morphology, etc. of various features of the respiratory cycles, including but not limited to the inspiratory phase, expiratory active phase, etc. In some examples, the respiration information excludes (i.e. is without) tracking a respiratory volume and/or respiratory rate. In some examples, stimulation based on such synchronization may be delivered throughout a treatment period or throughout substantially the entire treatment period. In some examples, such stimulation may be delivered just during a portion or portions of a treatment period.

In some examples of "synchronization", synchronization of the stimulation relative to the inspiratory phase may extend to a pre-inspiratory period and/or a post-inspiratory phase. For instance, in some such examples, a beginning of the synchronization may occur at a point in each respiratory cycle which is just prior to an onset of the inspiratory phase. In some examples, this point may be about 200 milliseconds, or 300 milliseconds prior to an onset of the inspiratory phase.

In some examples in which the stimulation is synchronous with at least a portion of the inspiratory phase, the upper airway muscles are contracted via the stimulation to ensure they are open at the time the respiratory drive controlled by the central nervous system initiates an inspiration (inhalation). In some such examples, in combination with the stimulation occurring during the inspiratory phase, example implementation of the above-noted pre-inspiratory stimulation helps to ensure that the upper airway is open before the negative pressure of inspiration within the respiratory system is applied via the diaphragm of the patient's body. In one aspect, this example arrangement may minimize the chance of constriction or collapse of the upper airway, which might otherwise occur if flow of the upper airway flow were too limited prior to the full force of inspiration occurring.

In some such examples, the stimulation of the upper airway patency-related nerve may be synchronized to occur with at least a portion of the expiratory period.

With regard to at least the methods of treating sleep apnea as previously described in association with at least FIGS. 1-22, at least some such methods may comprise performing the delivery of stimulation to the upper airway patency-related first nerve without synchronizing such stimulation relative to a portion of a respiratory cycle. In some instances, such methods may sometimes be referred to as the previously described open loop stimulation.

In some examples, the term "without synchronizing" may refer to performing the stimulation independently of timing of a respiratory cycle. In some examples, the term "without synchronizing" may refer to performing the stimulation while being aware of respiratory information but without necessarily triggering the initiation of stimulation relative to a specific portion of a respiratory cycle or without causing the stimulation to coincide with a specific portion (e.g. inspiratory phase) of respiratory cycle.

In some examples, in this context the term "without synchronizing" may refer to performing stimulation upon the detection of sleep disordered breathing behavior (e.g. obstructive sleep apnea events) but without necessarily triggering the initiation of stimulation relative to a specific portion of a respiratory cycle or without causing the stimulation to coincide with the inspiratory phase. At least some such examples may be described in Wagner et al. WO 2016/149344, STIMULATION FOR TREATING SLEEP DISORDERED BREATHING, published September 22, 2016.

In some examples, while open loop stimulation may be performed continuously without regard to timing of respiratory information (e.g. inspiratory phase, expiratory phase, etc.) such an example method and/or system may still comprise sensing respiration information for diagnostic data and/or to determine whether (and by how much) the continuous stimulation should be adjusted. For instance, via such respiratory sensing, it may be determined that the number of sleep disordered breathing (SDB) events are too numerous (e.g. an elevated AHI) and therefore the intensity (e.g. amplitude, frequency, pulse width, etc.) of the continuous stimulation should be increased or that the SDB events are relative low such that the intensity of the continuous stimulation can be decreased while still providing therapeutic stimulation. It will be understood that via such respiratory sensing, other SDB-related information may be determined which may be used for diagnostic purposes and/or used to determine adjustments to an intensity of stimulation, initiating stimulation, and/or terminating stimulation to treat sleep disordered breathing. It will be further understood that such "continuous" stimulation may be implemented via selectable duty cycles, train of stimulation pulses, selective activation of different combinations of electrodes, etc.

In some examples of open loop stimulation or closed loop stimulation, some sensory feedback may be utilized to determine, in general, whether the patient should receive stimulation based on a severity of sleep apnea behavior. In other words, upon sensing that a certain number of sleep apnea events are occurring, the device may implement stimulation.

Some non-limiting examples of such devices and methods to recognize and detect the various features and patterns associated with respiratory effort and flow limitations include, but are not limited to: PCT Publication WO/2010/059839, titled A METHOD OF TREATING SLEEP APNEA, published on May 27, 2010; Christopherson U.S. Patent 5,944,680, titled RESPIRATORY EFFORT DETECTION METHOD AND APPARATUS; and Testerman U.S. Patent 5,522,862, titled METHOD AND APPARATUS FOR TREATING OBSTRUCTIVE SLEEP APNEA.

Moreover, in some examples various stimulation methods may be applied to treat obstructive sleep apnea, which include but are not limited to: Ni et al. WO 2013/023218, SYSTEM FOR SELECTING A STIMULATION PROTOCOL BASED ON SENSED RESPIRATORY EFFORT; Christopherson et al. US 8938299, SYSTEM FOR TREATING SLEEP DISORDERED BREATHING, issued January 20, 2015; and Wagner et al. WO 2016/149344, STIMULATION FOR TREATING SLEEP DISORDERED BREATHING, published September 22, 2016.

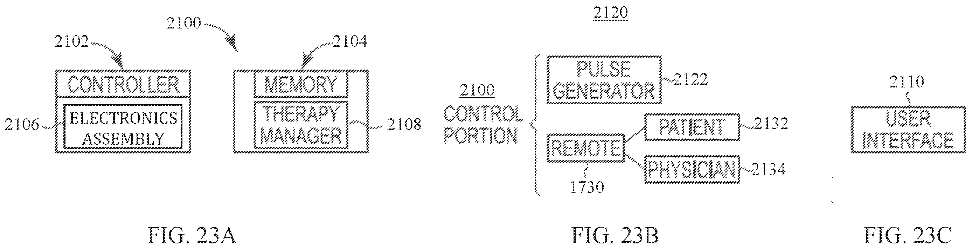

As implicated by the above descriptions, the stimulation system 1910 includes a controller, control unit, or control portion that prompts performance of designated actions. FIG. 23A is a block diagram schematically representing a control portion 2100, according to one example of the present disclosure. In some examples, the control portion 2100 includes a controller 2102 and a memory 2104. In some examples, the control portion 2100 provides one example implementations of a control portion forming a part of, implementing, and/or managing any one of devices, systems, assemblies, circuitry, managers, engines, functions, parameters, sensors, electrodes, modules, and/or methods, as represented throughout the present disclosure.

In general terms, the controller 2102 of the control portion 2100 comprises an electronics assembly 2106 (e.g., at least one processor, microprocessor, integrated circuits and logic, etc.) and associated memories or storage devices. The controller 2102 is electrically couplable to, and in communication with, the memory 2104 to generate control signals to direct operation of at least some the devices, systems, assemblies, circuitry, managers, modules, engines, functions, parameters, sensors, electrodes, and/or methods, as represented throughout the present disclosure can be a software program stored on a storage device, loaded onto the memory 2104, and executed by the electronics assembly 2106. In addition, and in some examples, these generated control signals include, but are not limited to, employing a therapy manager 2108 stored in the memory 2104 to at least manage therapy delivered to the patient, for example therapy for sleep disordered breathing, and/or manage and operate designated sensing in the manner described in at least some examples of the present disclosure. It will be further understood that the control portion 2100 (or another control portion) may also be employed to operate general functions of the various therapy devices/systems described throughout the present disclosure.

In response to or based upon commands received via a user interface (e.g. user interface 2110 in FIG. 23C) and/or via machine readable instructions, the controller 2102 generates control signals to implement therapy implementation, therapy monitoring, therapy management, and/or management and control in accordance with at least some of the previously described examples of the present disclosure. In some examples, the controller 2102 is embodied in a general purpose computing device while in some examples, the controller 2102 is incorporated into or associated with at least some of the associated devices, systems, assemblies, circuitry, sensors, electrodes, components of the devices and/or managers, engines, parameters, functions etc. described throughout the present disclosure.

For purposes of the present disclosure, in reference to the controller 2102, with embodiments in which the electronics assembly 2106 comprises or includes at least one processor, the term "processor" shall mean a presently developed or future developed processor (or processing resources) or microprocessor that executes sequences of machine readable instructions contained in a memory. In some examples, execution of the sequences of machine readable instructions, such as those provided via the memory 2104 of the control portion 2100 cause the processor to perform actions, such as operating the controller 2102 to implement sleep disordered breathing (SDS) therapy and related management as generally described in (or consistent with) at least some examples of the present disclosure. The machine readable instructions may be loaded in a random access memory (RAM) for execution by the processor from their stored location in a read only memory (ROM), a mass storage device, or some other persistent storage (e.g., non-transitory tangible medium or non-volatile tangible medium, as represented by the memory 2104. In some examples, the memory 2104 comprises a computer readable tangible medium providing non-volatile storage of the machine readable instructions executable by a process of the controller 2102. In other examples, hard wired circuitry may be used in place of or in combination with machine readable instructions to implement the functions described. For example, the electronics assembly 2106 may be embodied as part of at least one application-specific integrated circuit (ASIC), at least one integrated circuit, a microprocessor and ASIC, etc. In at least some examples, the controller 2102 is not limited to any specific combination of hardware circuitry and machine readable instructions, nor limited to any particular source for the machine readable instructions executed by the controller 2102.

FIG. 23B is a diagram 2120 schematically illustrating at least some manners in which the control portion 2100 can be implemented, according to one example of the present disclosure. In some examples, the control portion 2100 is entirely implemented within or by an IPG assembly 2122, which has at least some of substantially the same features and attributes as the IPG assembly 1912 as previously described in association with at least FIG. 22. In some examples, the control portion 2100 is entirely implemented within or by a remote control 2130 (e.g. a programmer) external to the patient's body, such as a patient control 2132 and/or a physician control 2134. In some examples, the control portion 2100 is partially implemented in the IPG assembly 2122 and partially implemented in the remote control 2130 (at least one of the patient control 2132 and the physician control 2134). In some examples the control portion 2100 may be implemented via a server accessible via the cloud and/or other network pathways. In some examples, the control portion 2100 may be distributed or apportioned among multiple devices or resources such as among a server, an IMD, and/or a user interface

In some examples, in association with the control portion 2100, the user interface (2110 in FIG. 23C) is implemented in the remote control 2130. FIG. 23C is a block diagram schematically representing the user interface 2110, according to one example of the present disclosure. In some examples, the user interface 2110 forms part of and/or is accessible via a device external to the patient and by which the therapy system may be at least partially controlled and/or monitored. The external device hosting the user interface 2110 may be a patient remote (e.g., 2132 in FIG. 22C, for example a smartphone operating a custom software application), a physician remote (e.g., 2134 in FIG. 23B) and/or a clinician portal. In some examples, the user interface 2110 comprises a user interface or other display that provides for the simultaneous display, activation, and/or operation of at least some of the various systems, assemblies, circuitry, engines, sensors, components, modules, functions, parameters, as described in the present disclosure. In some examples, at least some portions or aspects of the user interface 2110 are provided via a graphical user interface (GUI), and may comprise a display and input.

Although specific examples have been illustrated and described herein, a variety of implementations which fall under the scope of the claims may be used.

## Claims

1. A system (1910) for treating sleep disordered breathing (SDB) in a patient, comprising:
an implantable stimulation electrode assembly (1900) comprising:
a support body (1920) defining opposing, first and second ends, a central major axis and a central minor axis perpendicular to the central major axis and midway between the opposing ends;
a first stimulation electrode (1922) carried by the support body and located between the central minor axis and the first end;
a second stimulation electrode (1922) carried by the support body and located between the central minor axis and the second end;
wherein the support body is configured for implantation across a mid-line of a chin of the patient, including the first and second stimulation electrodes located at opposite sides of the mid-line; **characterized in that** it further comprises
a lead body (1970) coupled to the support body, with the support body being arranged relative to the lead body such that the second end is proximate the lead body and the first end is opposite the lead body.

2. The system (1910) of claim 1, wherein the implantable stimulation electrode assembly is implantable via insertion through an incision at the chin of the patient at a location lateral to the mid-line.

3. The system (1910) of claim 1, wherein the support body (1920) has an elongated shape.

4. The system (1910) of claim 1, wherein the first and second stimulating electrodes (1922) each have an axially symmetric shape.

5. The system (1910) of claim 1, wherein the first stimulation electrode (1922) is provided as one of a first sub-group of electrodes (1930) located between the central minor axis and the first end, and further wherein the second stimulation electrode (1922) is provided as one of a second sub-group of electrodes (1932) located between the central minor axis and the second end.

6. The system (1910) of claim 1, wherein the implantable stimulation electrode assembly (1900) is implantable to locate the first stimulation electrode (1922) in a vicinity of a medial branch of a right hypoglossal nerve of the patient and the second stimulation electrode (1922) in a vicinity of a medial branch of a left hypoglossal nerve of the patient, wherein the first stimulation electrode (1922) is one of a first sub-group of electrodes (1930) and the second stimulation electrode (1922) is one of a second sub-group of electrodes (1932), the system further comprising an implantable pulse generator (IPG) (1912) programmed to deliver stimulation energy to the stimulation electrodes (1922) of the first and second sub-groups based upon a location of each of the stimulation electrodes (1922) relative to the medial branch of the right hypoglossal nerve and the medial branch of the left hypoglossal nerve.

7. The system (1910) of claim 1, further comprising an implantable pulse generator (IPG) (1912) programmed to deliver stimulation energy to at least one of the first and second stimulation electrodes (1922).

8. The system (1910) of claim 7, wherein the implantable stimulation electrode assembly (1900) is implantable to locate the first stimulation electrode (1922) in a vicinity of a right hypoglossal nerve of the patient and the second stimulation element (1922) in a vicinity of a left hypoglossal nerve of the patient, wherein the first stimulation electrode (1922) is one of a first sub-group of electrodes (1930) and the second stimulation electrode (1922) is one of a second sub-group of electrodes (1932), wherein the IPG (1912) is programmed to deliver stimulation to only one of the first and second sub-groups of electrodes to stimulate only one of the right and left hypoglossal nerves.

9. The system (1910) of claim 7, wherein the implantable stimulation electrode assembly (1900) is implantable to locate the first stimulation electrode (1922) in a vicinity of a right hypoglossal nerve of the patient and the second stimulation element (1922) in a vicinity of a left hypoglossal nerve of the patient, wherein the IPG (1912) is programmed to deliver stimulation energy to one or more of the stimulation electrodes of the first sub-group of electrodes and to deliver stimulation energy to one or more of the stimulation electrodes of the second sub-group of electrodes to stimulate both of the right and left hypoglossal nerves.

10. The system (1910) of claim 9, wherein the IPG (1912) is programmed to deliver a stimulation signal to the first stimulation electrode (1922) that differs from a stimulation signal delivered to the second stimulation electrode (1922) by at least one of amplitude, pulse width, and rate.

11. The system (1910) of claim 9, wherein the IPG (1912) is programmed to deliver interleaved pulse trains to the first and second stimulation electrodes (1922).

12. The system (1910) of claim 1, further comprising:
an introducer (1700, 1720) for guiding the stimulation electrode assembly (1900) through an incision in a skin of the patient.

13. The system (1910) of claim 12, wherein the system is configured to apply test stimulation energy at a preliminary implant location by at least one of:
a test electrode (1728) carried by the introducer (1720); and
at least one of the first and second stimulation electrodes (1922) of the stimulation electrode assembly (1900) through an open channel defined in the introducer (1700).

14. The system (1910) of claim 1, wherein the support body defines a curved shape in extension between the first and second ends.

15. The system (1910) of claim 1, wherein the support body has a pre-defined shape in extension between the first and second ends, the pre-defined shape being one of a U shape and a W shape.

## Patentansprüche

1. System (1910) zum Behandeln von schlafbezogenen Atmungsstörungen (SDB) bei einem Patienten, umfassend:
eine implantierbare Stimulationselektrodenanordnung (1900) umfassend:
einen Stützkörper (1920), der gegenüberliegende, erste und zweite Enden, eine zentrale Hauptachse und eine zentrale Nebenachse definiert, die senkrecht zu der zentralen Hauptachse und in der Mitte zwischen den gegenüberliegenden Enden verläuft;
eine erste Stimulationselektrode (1922), die durch den Stützkörper getragen wird und zwischen der zentralen Nebenachse und dem ersten Ende positioniert ist;
eine zweite Stimulationselektrode (1922), die durch den Stützkörper getragen wird und zwischen der zentralen Nebenachse und dem zweiten Ende positioniert ist;
wobei der Stützkörper für eine Implantation quer über eine Mittellinie eines Kinns des Patienten konfiguriert ist, einschließlich der ersten und der zweiten Stimulationselektrode, die auf gegenüberliegenden Seiten der Mittellinie positioniert sind; **dadurch gekennzeichnet, dass** es ferner umfasst
einen Schaftkörper (1970), der mit dem Stützkörper gekoppelt ist, wobei der Stützkörper relativ zu dem Schaftkörper derart eingerichtet ist, dass das zweite Ende in der Nähe des Schaftkörpers liegt und das erste Ende dem Schaftkörper gegenüberliegt.

2. System (1910) nach Anspruch 1, wobei die implantierbare Stimulationselektrodenanordnung über Einführung durch einen Einschnitt an dem Kinn des Patienten an einer Position seitlich der Mittellinie implantierbar ist.

3. System (1910) nach Anspruch 1, wobei der Stützkörper (1920) eine längliche Form aufweist.

4. System (1910) nach Anspruch 1, wobei die erste und die zweite Stimulationselektrode (1922) jeweils eine axialsymmetrische Form aufweisen.

5. System (1910) nach Anspruch 1, wobei die erste Stimulationselektrode (1922) als eine von einer ersten Untergruppe von Elektroden (1930) bereitgestellt ist, die zwischen der zentralen Nebenachse und dem ersten Ende positioniert ist, und ferner wobei die zweite Stimulationselektrode (1922) als eine von einer zweiten Untergruppe von Elektroden (1932) bereitgestellt ist, die zwischen der zentralen Nebenachse und dem zweiten Ende positioniert ist.

6. System (1910) nach Anspruch 1, wobei die implantierbare Stimulationselektrodenanordnung (1900) implantierbar ist, um die erste Stimulationselektrode (1922) in einer Nähe eines medialen Astes eines rechten Nervus hypoglossus des Patienten und die zweite Stimulationselektrode (1922) in einer Nähe eines medialen Astes eines linken Nervus hypoglossus des Patienten zu positionieren, wobei die erste Stimulationselektrode (1922) eine von einer ersten Untergruppe von Elektroden (1930) ist und die zweite Stimulationselektrode (1922) eine von einer zweiten Untergruppe von Elektroden (1932) ist, das System ferner umfassend einen implantierbaren Impulsgenerator (IPG) (1912), der programmiert ist, um Stimulationsenergie an die Stimulationselektroden (1922) der ersten und der zweiten Untergruppe abzugeben, basierend auf einer Position jeder der Stimulationselektroden (1922) relativ zu dem medialen Ast des rechten Nervus hypoglossus und dem medialen Ast des linken Nervus hypoglossus.

7. System (1910) nach Anspruch 1, ferner umfassend einen implantierbaren Impulsgenerator (IPG) (1912), der programmiert ist, um Stimulationsenergie an mindestens eine der ersten und der zweiten Stimulationselektrode (1922) abzugeben.

8. System (1910) nach Anspruch 7, wobei die implantierbare Stimulationselektrodenanordnung (1900) implantierbar ist, um die erste Stimulationselektrode (1922) in einer Nähe eines rechten Nervus hypoglossus des Patienten und das zweite Stimulationselement (1922) in einer Nähe eines linken Nervus hypoglossus des Patienten zu positionieren, wobei die erste Stimulationselektrode (1922) eine von einer ersten Untergruppe von Elektroden (1930) ist und die zweite Stimulationselektrode (1922) eine von einer zweiten Untergruppe von Elektroden (1932) ist, wobei der IPG (1912) programmiert ist, um Stimulation nur an eine der ersten und der zweiten Untergruppe von Elektroden abzugeben, um nur einen des rechten und des linken Nervus hypoglossus zu stimulieren.

9. System (1910) nach Anspruch 7, wobei die implantierbare Stimulationselektrodenanordnung (1900) implantierbar ist, um die erste Stimulationselektrode (1922) in einer Nähe eines rechten Nervus hypoglossus des Patienten und das zweite Stimulationselement (1922) in einer Nähe eines linken Nervus hypoglossus des Patienten zu positionieren, wobei der IPG (1912) programmiert ist, um Stimulationsenergie an eine oder mehrere der Stimulationselektroden der ersten Untergruppe von Elektroden und Stimulationsenergie an eine oder mehrere der Stimulationselektroden der zweiten Untergruppe von Elektroden abzugeben, um sowohl den rechten als auch den linken Nervus hypoglossus zu stimulieren.

10. System (1910) nach Anspruch 9, wobei der IPG (1912) programmiert ist, um ein Stimulationssignal an die erste Stimulationselektrode (1922) abzugeben, das sich von einem Stimulationssignal, das an die zweite Stimulationselektrode (1922) abgegeben wird, durch mindestens eines von Amplitude, Impulsbreite und Frequenz unterscheidet.

11. System (1910) nach Anspruch 9, wobei der IPG (1912) programmiert ist, um verschachtelte Impulsfolgen an die erste und die zweite Stimulationselektrode (1922) abzugeben.

12. System (1910) nach Anspruch 1, ferner umfassend:
ein Einführinstrument (1700, 1720) zum Leiten der Stimulationselektrodenanordnung (1900) durch einen Einschnitt in eine Haut des Patienten.

13. System (1910) nach Anspruch 12, wobei das System konfiguriert ist, um Teststimulationsenergie an einer vorläufigen Implantationsposition durch mindestens eines anzulegen von:
einer Testelektrode (1728), die durch das Einführinstrument (1720) getragen wird; und
mindestens einer der ersten und der zweiten Stimulationselektrode (1922) der Stimulationselektrodenanordnung (1900) durch einen in dem Einführinstrument (1700) definierten offenen Kanal.

14. System (1910) nach Anspruch 1, wobei der Stützkörper eine gekrümmte Form in einer Ausdehnung zwischen dem ersten und dem zweiten Ende definiert.

15. System (1910) nach Anspruch 1, wobei der Stützkörper eine vordefinierte Form in der Ausdehnung zwischen dem ersten und dem zweiten Ende aufweist, wobei die vordefinierte Form eine von einer U-Form und einer W-Form ist.

## Revendications

1. Système (1910) destiné à traiter un trouble respiratoire du sommeil (SDB) chez un patient, comprenant :
un jeu d'électrodes de stimulation (1900) implantable comprenant :
un corps de support (1920) définissant des première et deuxième extrémités opposées, un axe majeur central et un axe mineur central perpendiculaire à l'axe majeur central et à mi-chemin entre les extrémités opposées ;
une première électrode de stimulation (1922) portée par le corps de support et située entre l'axe mineur central et la première extrémité ;
une deuxième électrode de stimulation (1922) portée par le corps de support et située entre l'axe mineur central et la deuxième extrémité ;
dans lequel le corps de support est configuré pour une implantation en travers d'une ligne médiane d'un menton du patient, incluant les première et deuxième électrodes de stimulation situées en des côtés opposés de la ligne médiane ; **caractérisé en ce qu'**il comprend en outre un corps de tige (1970) couplé avec le corps de support, le corps de support étant agencé par rapport au corps de tige de sorte que la deuxième extrémité soit à proximité du corps de tige et que la première extrémité soit opposée au corps de tige.

2. Système (1910) selon la revendication 1, dans lequel le jeu d'électrodes de stimulation implantable peut être implanté via une insertion par le biais d'une incision au niveau du menton du patient en un emplacement latéral par rapport à la ligne médiane.

3. Système (1910) selon la revendication 1, dans lequel le corps de support (1920) présente une forme allongée.

4. Système (1910) selon la revendication 1, dans lequel les première et deuxième électrodes stimulantes (1922) présentent chacune une forme axialement symétrique.

5. Système (1910) selon la revendication 1, dans lequel la première électrode de stimulation (1922) est prévue en tant qu'une parmi un premier sous-groupe d'électrodes (1930) situées entre l'axe mineur central et la première extrémité, et en outre dans lequel la deuxième électrode de stimulation (1922) est prévue en tant qu'une parmi un deuxième sous-groupe d'électrodes (1932) situées entre l'axe mineur central et la deuxième extrémité.

6. Système (1910) selon la revendication 1, dans lequel le jeu d'électrodes de stimulation (1900) implantable peut être implanté de façon à situer la première électrode de stimulation (1922) dans un voisinage d'une branche médiane d'un nerf hypoglosse droit du patient et la deuxième électrode de stimulation (1922) dans un voisinage d'une branche médiane d'un nerf hypoglosse gauche du patient, dans lequel la première électrode de stimulation (1922) est l'une parmi un premier sous-groupe d'électrodes (1930) et la deuxième électrode de stimulation (1922) est l'une parmi un deuxième sous-groupe d'électrodes (1932), le système comprenant en outre un générateur d'impulsions implantable (IPG) (1912) programmé pour fournir une énergie de stimulation aux électrodes de stimulation (1922) des premier et deuxième sous-groupes d'électrodes sur la base d'un emplacement de chacune des électrodes de stimulation (1922) par rapport à la branche médiane du nerf hypoglosse droit et la branche médiane du nerf hypoglosse gauche.

7. Système (1910) selon la revendication 1, comprenant en outre un générateur d'impulsions implantable (IPG) (1912) programmé pour fournir une énergie de stimulation à au moins l'une parmi les première et deuxième électrodes de stimulation (1922).

8. Système (1910) selon la revendication 7, dans lequel le jeu d'électrodes de stimulation (1900) implantable peut être implanté de façon à situer la première électrode de stimulation (1922) dans un voisinage d'un nerf hypoglosse droit du patient et le deuxième élément de stimulation (1922) dans un voisinage d'un nerf hypoglosse gauche du patient, dans lequel la première électrode de stimulation (1922) est l'une parmi un premier sous-groupe d'électrodes (1930) et la deuxième électrode de stimulation (1922) est l'une parmi un deuxième sous-groupe d'électrodes (1932), dans lequel l'IPG (1912) est programmé pour fournir une stimulation à uniquement l'un parmi les premier et deuxième sous-groupe d'électrodes afin de stimuler uniquement l'un parmi les nerfs hypoglosses droit et gauche.

9. Système (1910) selon la revendication 7, dans lequel le jeu d'électrodes de stimulation (1900) implantable peut être implanté de façon à situer la première électrode de stimulation (1922) dans un voisinage d'un nerf hypoglosse droit du patient et le deuxième élément de stimulation (1922) dans un voisinage d'un nerf hypoglosse gauche du patient, dans lequel l'IPG (1912) est programmé pour fournir une énergie de stimulation à une ou plusieurs parmi les électrodes de stimulation du premier sous-groupe d'électrodes et pour fournir une énergie de stimulation à une ou plusieurs parmi les électrodes de stimulation du deuxième sous-groupe d'électrodes afin de stimuler les deux parmi les nerfs hypoglosses droit et gauche.

10. Système (1910) selon la revendication 9, dans lequel l'IPG (1912) est programmé pour fournir un signal de stimulation à la première électrode de stimulation (1922) qui diffère d'un signal de stimulation fourni à la deuxième électrode de stimulation (1922) d'au moins l'une parmi une amplitude, une largeur d'impulsion et une fréquence.

11. Système (1910) selon la revendication 9, dans lequel l'IPG (1912) est programmé pour fournir des trains d'impulsions imbriqués aux première et deuxième électrodes de stimulation (1922).

12. Système (1910) selon la revendication 1, comprenant en outre :
un introducteur (1700, 1720) pour guider le jeu d'électrodes de stimulation (1900) implantable par le biais d'une incision dans une peau du patient.

13. Système (1910) selon la revendication 12, dans lequel le système est configuré pour appliquer une énergie de stimulation de test en un emplacement d'implant préliminaire par au moins l'un parmi :
une électrode de test (1728) portée par l'introducteur (1720) ; et
au moins l'une parmi les première et deuxième électrodes de stimulation (1922) du jeu d'électrodes de stimulation (1900) par le biais d'un canal ouvert défini dans l'introducteur (1700).

14. Système (1910) selon la revendication 1, dans lequel le corps de support définit une forme courbe en extension entre les première et deuxième extrémités.

15. Système (1910) selon la revendication 1, dans lequel le corps de support présente une forme prédéfinie en extension entre les première et deuxième extrémités, la forme prédéfinie étant l'une parmi une forme en U et une forme en W.
